(19) **Europäisches Patentamt / European Patent Office / Office européen des brevets**

(11) **EP 4 364 718 A1**

(12) **EUROPEAN PATENT APPLICATION**
published in accordance with Art. 153(4) EPC

(43) Date of publication:
**08.05.2024 Bulletin 2024/19**

(21) Application number: **22833188.0**

(22) Date of filing: **28.06.2022**

(51) International Patent Classification (IPC):
**A61K 6/896** (2020.01)  **A61K 6/20** (2020.01)
**A61K 6/60** (2020.01)  **A61K 6/64** (2020.01)
**A61K 6/71** (2020.01)  **A61K 6/76** (2020.01)
**A61K 6/818** (2020.01)  **A61K 6/887** (2020.01)
**A61K 6/893** (2020.01)

(52) Cooperative Patent Classification (CPC):
**A61K 6/20; A61K 6/60; A61K 6/64; A61K 6/71;**
**A61K 6/76; A61K 6/818; A61K 6/887; A61K 6/893;**
**A61K 6/896**

(86) International application number:
**PCT/JP2022/025853**

(87) International publication number:
**WO 2023/277042 (05.01.2023 Gazette 2023/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.06.2021 JP 2021107966**

(71) Applicant: **Kuraray Noritake Dental Inc.
Kurashiki-shi, Okayama 710-0801 (JP)**

(72) Inventors:
• **SAKAMOTO, Hiroyuki**
  **Miyoshi-shi, Aichi 470-0293 (JP)**
• **KASHIKI, Nobusuke**
  **Miyoshi-shi, Aichi 470-0293 (JP)**
• **YOSHIDA, Miki**
  **Iwakura-shi, Aichi 482-8510 (JP)**
• **NOZAKI, Akira**
  **Iwakura-shi, Aichi 482-8510 (JP)**
• **HARA, Chihiro**
  **Iwakura-shi, Aichi 482-8510 (JP)**

(74) Representative: **D Young & Co LLP
3 Noble Street
London EC2V 7BQ (GB)**

(54) **DENTAL CURABLE COMPOSITION**

(57) The present invention provides a dental curable composition and a dental prosthesis therewith with which shade changes in the base material can be reduced, and that exhibit excellent gloss retention in the cured product while demonstrating excellence in operability. The present invention relates to a dental curable composition comprising a polymerizable compound (A), a polyfunctional thiol (B) having two or more mercapto groups per molecule, a polysiloxane (C), and a polymerization initiator (D).

EP 4 364 718 A1

**Description**

TECHNICAL FIELD

The present invention relates to dental curable compositions and dental prostheses. More specifically, the invention relates to a dental curable composition for coating base materials, and to a dental prosthesis comprising a base material and a resin layer.

BACKGROUND ART

In the past, metals were commonly used for dental prostheses (for example, such as veneer crowns, dental caps, crowns, and post crowns). However, metals had the drawback of lacking aesthetics, and occasionally causing allergic reactions due to leaching of metal. To address the issues associated with the use of metals, ceramic materials such as aluminum oxide (alumina), zirconium oxide (zirconia), fused quartz, and lithium silicate glass, as well as acrylic resins, and composite materials containing polymer resins and inorganic fillers have been used for dental products as alternatives to metal. Zirconia, in particular, offers superior aesthetics and strength, and has seen a rise in demand, especially due to its increasing affordability in recent years.

To achieve improved aesthetics in the oral cavity, it is necessary for dental prostheses to mimic the look of natural teeth. However, achieving a natural tooth-like appearance, especially in terms of transparency, gloss (glaze or shine), and shade, is difficult with zirconia (a sintered body) alone. This issue is addressed by a veneer crown (see, for example, Patent Literature 3) aiming to replicate the appearance of natural teeth by baking a ceramic material called porcelain onto the exposed surface of a zirconia frame (base material), rather than leaving the zirconia exposed (see, for example, Patent Literature 1 and Patent Literature 2). Such dental products are called porcelain-fused-to-zirconia (PFZ) crowns.

In another technique that can achieve an aesthetic appearance similar to natural teeth, an adhesive is applied to a high-strength ceramic frame used as a base material, and a polymerizable composition, primarily composed of a polymerizable monomer, an inorganic filler, and a polymerization catalyst (commonly known as a dental composite resin) is built up in layers and allowed to polymerize and cure. For example, there are reports of aesthetic dental prostheses or methods for protecting the tooth structure. Patent Literature 1 uses a polymerizable monomer to coat zirconia dental prostheses. Patent Literature 2 builds up a polymerizable composition (dental composite resin) on a ceramic frame (base material). In Patent Literature 3, a coating is formed with a composition that comprises a (meth)acrylate monomer containing inorganic fine particles that have been surface-modified with alkoxysilane having unsaturated double bonds. In Patent Literature 4, a polymerizable monomer containing an $\alpha$-diketone compound and an amine compound is coated before it is polymerized and cured. Additionally, Patent Literature 5 discloses a dental curable composition comprising a polymerizable monomer, an inorganic filler, a polyorganosilsesquioxane particle, and a polymerization initiator, demonstrating excellence in gloss polishability.

CITATION LIST

Patent Literature

Patent Literature 1: JP 2018-89312 A
Patent Literature 2: JP 2001-149385 A
Patent Literature 3: JP 2005-154312 A
Patent Literature 4: JP 2021-54813 A
Patent Literature 5: JP 2016-153382 A

SUMMARY OF INVENTION

Technical Problem

Typically, PFZ is created by applying a slurry containing ceramic materials, which transform into porcelain, onto a base material, followed by firing at temperatures of several hundred degrees Celsius to fuse the porcelain to the base material. To prevent defects during firing, it is therefore required to choose a porcelain material with a coefficient of thermal expansion similar to that of the base material. For example, when a zirconia sintered body is used as the base material, it is essential to choose a porcelain ceramic material with a coefficient of thermal expansion close to that of the zirconia sintered body. Additionally, in the process of PFZ fabrication, it has been required to heat the porcelain to a temperature at which it melts (typically ranging from about 900 to 1,000°C) using an electric furnace, after applying a porcelain slurry to the zirconia surface. There accordingly is a need for a more convenient method to produce aesthetically pleasing zirconia prostheses without using porcelain.

Patent Literature 1 discloses a method for enhancing the translucency of base material zirconia with a prosthesis obtained by coating a polymerizable monomer. Patent Literature 2 discloses a dental prosthesis produced by building up a polymerizable composition on an alumina base material. However, with the polymerizable monomers disclosed, both techniques face a challenge in maintaining sufficient durability to sustain gloss in the demanding environment of the oral cavity. Another issue is that Patent Literature 1 involves a possible shift in the shade of the resin layer coating the base material due to polymerization and cure, though the method provides satisfactory translucency. That is, there is room for improvement in reducing shade changes in coating the base material, in addition to providing transparency.

The photopolymerizable dental coating material composition described in Patent Literature 3 involves an issue of undergoing gelation during storage, resulting in decline in surface curability over time. The photopolymerizable dental coating material composition described in Patent Literature 4 faces a challenge in maintaining sufficient durability to sustain gloss in the demanding environment of the oral cavity, and no consideration is given concerning ceramic materials. The dental curable composition described in Patent Literature 5 excels in properties such as transparency. However, there is still room for improvement in glossiness to achieve an appearance comparable to natural teeth, as well as in the operability in building up the dental curable composition.

It is an object of the present invention to provide a dental curable composition and a dental prosthesis therewith with which shade changes in the base material can be reduced, and that exhibit excellent gloss retention in the cured product while demonstrating excellence in operability.

Solution to Problem

The present inventors conducted intensive studies to find a solution to the foregoing issues, and found that, in dental curable compositions, combining a polysiloxane (C) with other components enables the composition to chemically and mechanically adhere to the base material, and form a coating layer maintaining appropriate levels of hardness, glossiness, and light reflection, providing a convenient way of retaining a natural gloss in dental prostheses for extended time periods. Further research based on this finding led to the completion of the present invention. Another finding is that a dental prosthesis including a base material and a resin layer is useful when the resin layer comprises a polymerized and cured product of a curable composition containing a polymerizable compound (A), a polyfunctional thiol (B), a polysiloxane (C), and a polymerization initiator (D), and covers the base material in a certain thickness. This finding prompted additional research, resulting in the completion of the present invention.

Specifically, the present invention relates to the following.

[1] A dental curable composition comprising a polymerizable compound (A), a polyfunctional thiol (B) having two or more mercapto groups per molecule, a polysiloxane (C), and a polymerization initiator (D).

[2] The dental curable composition according to [1], wherein the polysiloxane (C) comprises a polysilsesquioxane (C-1).

[3] The dental curable composition according to [1] or [2], wherein the polymerizable compound (A) comprises a urethane (meth)acrylate oligomer (A-1), and/or a polymerizable monomer (A-2).

[4] The dental curable composition according to any one of [1] to [3], wherein the polyfunctional thiol (B) comprises a polyfunctional thiol having 2 to 4 mercapto groups per molecule.

[5] The dental curable composition according to any one of [1] to [4], wherein the mercapto groups in the polyfunctional thiol (B) are bonded to secondary carbon atoms or tertiary carbon atoms.

[6] The dental curable composition according to any one of [1] to [5], wherein the polysiloxane (C) is liquid at 25°C.

[7] The dental curable composition according to any one of [1] to [6], wherein the polymerization initiator (D) comprises a photopolymerization initiator (D-1).

[8] The dental curable composition according to any one of [1] to [7], which further comprises a filler (E).

[9] The dental curable composition according to [8], wherein the filler (E) comprises a hydrophobic silica (E-1).

[10] The dental curable composition according to [9], wherein the hydrophobic silica (E-1) has an average primary particle diameter of 5 to 100 nm.

[11] The dental curable composition according to any one of [1] to [10], wherein the dental curable composition is a coating agent for coating a base material.

[12] The dental curable composition according to [11], wherein the base material comprises at least one selected from the group consisting of zirconia, alumina, fused quartz, lithium silicate glass, an acrylic resin, and a composite material containing a polymer resin and an inorganic filler.

[13] The dental curable composition according to [12], wherein the zirconia comprises zirconia, and a stabilizing agent capable of preventing a phase transformation of zirconia.

[14] A dental prosthesis comprising a base material and a resin layer, wherein the resin layer comprises a polymerized and cured product of a dental curable composition comprising a polymerizable compound (A), a polyfunctional thiol (B) having two or more mercapto groups per molecule, a polysiloxane (C), and a polymerization initiator (D).

[15] The dental prosthesis according to [14], wherein the base material comprises at least one selected from the group consisting of zirconia, alumina, fused quartz, lithium silicate glass, an acrylic resin, and a composite material containing a polymer resin and an inorganic filler.

[16] The dental prosthesis according to [15], wherein the zirconia comprises zirconia, and a stabilizing agent capable of preventing a phase transformation of zirconia.

[17] The dental prosthesis according to any one of [14] to [16], wherein the resin layer has a thickness of 1 $\mu$m to 500 $\mu$m.

[18] The dental prosthesis according to any one of [14] to [17], wherein the polysiloxane (C) comprises a polysilsesquioxane (C-1).

[19] The dental prosthesis according to any one of [14] to [18], wherein the polymerizable compound (A) comprises a urethane (meth)acrylate oligomer (A-1), and/or a polymerizable monomer (A-2).

[20] The dental prosthesis according to any one of [14] to [19], wherein the polyfunctional thiol (B) comprises a polyfunctional thiol having 2 to 4 mercapto groups per molecule.

[21] The dental prosthesis according to any one of [14] to [20], wherein the mercapto groups in the polyfunctional thiol (B) are bonded to secondary carbon atoms or tertiary carbon atoms.

[22] The dental prosthesis according to any one of [14] to [21], wherein the polysiloxane (C) is liquid at 25°C.

[23] The dental prosthesis according to any one of [14] to [22], wherein the polymerization initiator (D) comprises a photopolymerization initiator (D-1).

[24] The dental prosthesis according to any one of [14] to [23], wherein the resin layer further comprises a filler (E).

[25] The dental prosthesis according to [24], wherein the filler (E) comprises a hydrophobic silica (E-1).

[26] The dental prosthesis according to [25], wherein the hydrophobic silica (E-1) has an average primary particle diameter of 5 to 100 nm.

Advantageous Effects of Invention

The present invention can provide a dental curable composition and a dental prosthesis therewith with which shade changes in the base material can be reduced, and that exhibit excellent gloss retention in the cured product while demonstrating excellence in operability.

The present invention demonstrates excellence in the curability of the dental curable composition, and enables the dental curable composition to be polymerized and cured in a short time period.

According to the present invention, a dental curable composition and a dental prosthesis can be provided that include a surface-coating resin layer exhibiting appropriate levels of hardness, and chemically and mechanically adhering to the base material, enabling improvement of abrasion resistance while protecting the opposing natural tooth (opposing tooth), or the prosthesis or base material itself, from wear, and sustaining gloss for extended time periods.

DESCRIPTION OF EMBODIMENTS

The following describes each component of a dental curable composition of the present invention in detail. It should be noted that, in this specification, "ordinary temperature" means 25°C, strictly speaking.

<Polymerizable Compound (A)>

The polymerizable compound (A) included in the present invention is described first. The polymerizable compound (A) included in the present invention preferably comprises a urethane (meth)acrylate oligomer (A-1). The urethane (meth)acrylate oligomer (A-1) is used to impart hardness and toughness to a cured product of a dental curable composition of the present invention. The urethane (meth)acrylate oligomer (A-1) also contributes to the curability and toughness of the cured product when combined with the polymerizable monomer (A-2) described below.

In this specification, compounds having a polymerizable group (for example, such as a vinyl group, a (meth)acryloyloxy group, or a (meth)acrylamide group), and having two or more mercapto groups per molecule are considered to be included in polyfunctional thiol (B).

In this specification, compounds that are polymers where silicon atoms form bonds with each other through oxygen atoms, and where at least some of the silicon atoms are linked to organic groups, and that possess polymerizable groups are considered to be included in polysiloxane (C).

In this specification, "(meth)acryl" means both methacryl and acryl, and "(meth)acryloyl" means both methacryloyl and acryloyl.

The urethane (meth)acrylate oligomer (A-1) used in a dental curable composition of the present invention is an oligomer having a urethane functional group -NHC(O)O-, and may have at least one structure, per molecule, selected from the group consisting of a polyester, a polyether, a polycarbonate, a polyurethane, and a poly-conjugated diene.

In view of the curability, viscosity, and coatability of the composition, the urethane (meth)acrylate oligomer (A-1) has a viscosity at 25°C of preferably 4,000 to 250,000 mPas, more preferably 6,000 to 200,000 mPas, even more preferably 8,000 to 150,000 mPas. The viscosity can be measured with, for example, a B-type viscometer (Brookfield viscometer) under 25°C, 20 rpm conditions.

In view of the hardness of the composition, a cured film of the urethane (meth)acrylate oligomer (A-1) has a pencil hardness of preferably F or higher, more preferably H or higher, even more preferably 2H or higher.

The pencil hardness of the cured film can be measured following, for example, JIS K 5600-5-4:1999.

In view of providing the preferred curability, viscosity, and hardness when combined with the polymerizable monomer (A-2) described below, commercially available products may be used for the urethane (meth)acrylate oligomer (A-1). Examples of such urethane (meth)acrylate oligomers (A-1) are as follows.

Urethane acrylate oligomers EBECRYL 8807, EBECRYL 8465, EBECRYL 8800, EBECRYL 4101, EBECRYL 4201, and KRM 7735 commercially available from DAICEL-ALLNEX LTD.;

Urethane acrylate oligomers UV-1700B and UV-6300B (pencil hardness of cured film: 2H to 3H), UV-7550B (pencil hardness of cured film: F), UV-7600B (pencil hardness of cured film: 3H), UV-7605B (pencil hardness of cured film: 3H to 4H), UV-7630B (pencil hardness of cured film: 3H), UV-7640B (pencil hardness of cured film: 3H to 4H), and UV-7650B (pencil hardness of cured film: 2H) commercially available from Mitsubishi Chemical Corporation;
Urethane acrylate oligomer CN9006NS commercially available from Sartomer Company, Exton, PA;
Pentaerythritol triacrylate hexamethylene diisocyanate urethane prepolymer UA-306H, pentaerythritol triacrylate toluene diisocyanate urethane prepolymer UA-306T, pentaerythritol triacrylate isophorone diisocyanate urethane prepolymer UA-306I, and dipentaerythritol pentaacrylate hexamethylene diisocyanate urethane prepolymer UA-510H commercially available from Kyoeisha Chemical Co., Ltd.; and
Urethane acrylate oligomers UN-2600, UN-6202, and UN-6304 (urethane acrylate oligomers with polyether skeletons) commercially available from Negami Chemical Industrial Co., Ltd.

Preferred among these examples are urethane acrylate oligomer UV-1700B (viscosity at 25°C: 40,000 to 100,000 mPa·s; pencil hardness of cured film: 4H), pentaerythritol triacrylate hexamethylene diisocyanate urethane prepolymer UA-306H (viscosity at 25°C: 10,000 to 40,000 mPa s; pencil hardness of cured film: 6H), and pentaerythritol triacrylate toluene diisocyanate urethane prepolymer UA-306T (viscosity at 25°C: 10,000 to 40,000 mPa·s; pencil hardness of cured film: 5H). These may be used alone, or two or more thereof may be used in combination. It is also possible to use a polymer of mixtures of these.

In view of curability, hardness, viscosity, and coatability, the content of urethane (meth)acrylate oligomer (A-1) is preferably 25 to 55 mass%, more preferably 30 to 50 mass%, even more preferably 35 to 45 mass% in a total amount of the dental curable composition. In certain embodiments, the content of urethane (meth)acrylate oligomer (A-1) is preferably 15 to 80 parts by mass, more preferably 20 to 75 parts by mass, even more preferably 25 to 70 parts by mass in total 100 parts by mass of polymerizable compound (A).

The polymerizable compound (A) included in the present invention preferably comprises a polymerizable monomer (A-2). In the present invention, the polymerizable monomer (A-2) is used to enhance curability, and adhesive properties to the base material. The polymerizable monomer (A-2) also contributes to the curability of the cured product when combined with the urethane (meth)acrylate oligomer (A-1) described above.

The polymerizable monomer (A-2) may be any known polymerizable monomer used for dental compositions. Specific examples of the polymerizable monomer (A-2) include esters of acids such as $\alpha$-cyanoacrylic acid, (meth)acrylic acid, $\alpha$-halogenated acrylic acid, crotonic acid, cinnamic acid, sorbic acid, maleic acid, and itaconic acid; and (meth)acrylamides, (meth)acrylamide derivatives, vinyl esters, vinyl ethers, mono-N-vinyl derivatives, and styrene derivatives. Particularly preferred for use are (meth)acrylic acid esters or (meth)acrylamides.

Examples of the (meth)acrylic acid ester or (meth)acrylamide polymerizable monomers include monofunctional monomers such as monofunctional (meth)acrylates, and monofunctional (meth)acrylamides, and polyfunctional monomers such as bifunctional (meth)acrylates, and tri- and higher functional (meth)acrylates (preferably, polyfunctional (meth)acrylate monomers). Preferred examples of the polymerizable monomers are as follows.

Monofunctional (meth)acrylate monomers and monofunctional (meth)acrylamide monomers (a-1)

Isobutyl (meth)acrylate, benzyl (meth)acrylate, lauryl (meth)acrylate, 2-(N,N-dimethylamino)ethyl (meth)acrylate, 2,3-dibromopropyl (meth)acrylate, 2-hydroxyethyl (meth)acrylate, 6-hydroxyhexyl (meth)acrylate, 10-hydroxydecyl (meth)acrylate, propylene glycol mono(meth)acrylate, glycerin mono(meth)acrylate, erythritol mono(meth)acrylate, N-methylol (meth)acrylamide, N-hydroxyethyl (meth)acrylamide, N,N-bis(2-hydroxyethyl) (meth)acrylamide, (meth)acryloyloxydodecylpyridinium bromide, (meth)acryloyloxydodecylpyridinium chloride, (meth)acryloyloxyhexadecylpyridinium chloride, and (meth)acryloyloxydecylammonium chloride.

Bifunctional (meth)acrylate monomers (a-2)

Ethylene glycol di(meth)acrylate, triethylene glycol di(meth)acrylate, propylene glycol di(meth)acrylate, neopentyl glycol di(meth)acrylate, 1,6-hexanediol di(meth)acrylate, 1,10-decanediol di(meth)acrylate, bisphenol A diglycidyl acrylate (2,2-bis[4-[3-acryloyloxy-2-hydroxypropoxy]phenyl]propane), bisphenol A diglycidyl methacrylate (2,2-bis[4-[2-hydroxy-3-methacryloyloxypropoxy]phenyl]propane, commonly known as Bis-GMA), 2,2-bis[4-(meth)acryloyloxyethoxyphenyl]propane, 2,2-bis[4-acryloyloxypolyethoxyphenyl]propane, 2,2-bis[4-methacryloyloxypolyethoxyphenyl]propane (with an average of 2.6 moles of ethoxy group added), 1,2-bis[3-(meth)acryloyloxy-2-hydroxypropoxy]ethane, pentaerythritol di(meth)acrylate, [2,2,4-trimethylhexamethylenebis(2-carbamoyloxyethyl)]di(meth)acrylate, and propoxylated (2)neopentyl glycol di(meth)acrylate.

Tri- and higher functional (meth)acrylate monomers (a-3)

Trimethylolpropane tri(meth)acrylate, trimethylolethane tri(meth)acrylate, tetramethylolmethane tri(meth)acrylate, pentaerythritol tri(meth)acrylate, pentaerythritol tetra(meth)acrylate, dipentaerythritol hexa(meth)acrylate, dipentaerythritol penta(meth)acrylate, ethoxylated (3)trimethylolpropane tri(meth)acrylate, propoxylated (3)trimethylolpropane tri(meth)acrylate, N,N-(2,2,4-trimethylhexamethylene)bis[2-(aminocarboxy)propane-1,3-diol]tetra(meth)acr ylate, and 2,2,6,6-tetrakis(acryloyloxymethyl)-4-oxaheptane-1,7-diol 1-(meth)acrylate.

In view of curability, preferred as the polymerizable monomer (A-2) are tri- and higher functional (meth)acrylate monomers (a-3). In view of ease of handling and safety, more preferred are pentaerythritol tri(meth)acrylate, pentaerythritol tetra(meth)acrylate, dipentaerythritol hexa(meth)acrylate, dipentaerythritol penta(meth)acrylate, and ethoxylated (3)trimethylolpropane tri(meth)acrylate.

The polymerizable monomer (A-2) may be incorporated alone, or two or more thereof may be incorporated in combination. For example, the polyfunctional (meth)acrylate monomers (bifunctional (meth)acrylate monomer (a-2), tri- and higher functional (meth)acrylate monomer (a-3)) may be incorporated alone, or two or more thereof may be incorporated in combination.

In view of curability, hardness, viscosity, and coatability, the content of polymerizable monomer (A-2) is preferably 20 to 55 mass%, more preferably 25 to 50 mass%, even more preferably 30 to 45 mass% in a total amount of the dental curable composition.

The content of polymerizable monomer (A-2) is preferably 15 to 80 parts by mass, more preferably 20 to 75 parts by mass, even more preferably 25 to 70 parts by mass in total 100 parts by mass of polymerizable compound (A).

A certain preferred embodiment is, for example, a dental curable composition in which the polymerizable compound (A) comprises a polymerizable monomer (A-2), and the polymerizable monomer (A-2) comprises an adhesive monomer (a-4) having at least one acidic group per molecule (hereinafter, also referred to as "adhesive monomer (a-4) having an acidic group"). With an adhesive monomer (a-4) having an acidic group, a stronger bond can be formed at the interface between the base material and the cured product of the composition forming a resin layer built up on the base material.

A dental adhesive composition (F) comprising an adhesive monomer (a-4) having an acidic group (hereinafter, referred to as "adhesive composition (F)"), different from a dental curable composition of the present invention, may be used with a dental curable composition of the present invention. A certain preferred embodiment is, for example, a dental kit that comprises a dental curable composition of the present invention, and a dental adhesive composition (F) comprising an adhesive monomer (a-4) having an acidic group. The dental curable composition of the present invention used for the dental kit may or may not comprise an adhesive monomer (a-4) having an acidic group. In the following, a dental adhesive composition (F) will be called a "primer composition (F)" when it contains a volatile organic solvent (G) in addition to the adhesive monomer (a-4) having an acidic group. The dental adhesive composition (F) used for the dental kit may be a commercially available product (for example, such as Clearfil® Ceramic® Primer Plus manufactured by Kuraray Noritake Dental Inc. under this trade name). The adhesive composition (F) comprising an adhesive monomer (a-4) having an acidic group enables formation of a stronger bond at the interface between the base material and the cured product of the composition forming a resin layer built up on the base material.

The adhesive monomer (a-4) having an acidic group is a polymerizable monomer having an acidic group such as a phosphoric acid group, a phosphonic acid group, a pyrophosphoric acid group, a thiophosphoric acid group, a carboxylic acid group, or a sulfonic acid group. Specific examples are as follows.

Examples of the phosphoric acid group-containing polymerizable monomer include 2-(meth)acryloyloxyethyl dihydrogen phosphate, 10-(meth)acryloyloxydecyl dihydrogen phosphate, 20-(meth)acryloyloxyeicosyl dihydrogen phosphate, 1,3-di(meth)acryloyloxypropyl-2-dihydrogen phosphate, 2-(meth)acryloyloxyethylphenyl phosphoric acid, 2-(meth)acryloyloxyethyl-2'-bromoethyl phosphoric acid, (meth)acryloyloxyethylphenyl phosphate, and acid chlorides of these.

Examples of the phosphonic acid group-containing polymerizable monomer include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexylphosphonoacetate, 10-(meth)acryloyloxydecylphosphonoacetate, and acid chlorides of these.

Examples of the pyrophosphoric acid group-containing polymerizable monomer include di[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides of these.

Examples of the thiophosphoric acid group-containing polymerizable monomer include 2-(meth)acryloyloxyethyl dihydrogen dithiophosphate, 10-(meth)acryloyloxydecyl dihydrogen thiophosphate, and acid chlorides of these.

Examples of the carboxylic acid group-containing polymerizable monomer include (meth)acrylic acid, mono(2-(meth)acryloyloxyethyl)succinate, mono(2-(meth)acryloyloxyethyl)isophthalate, N-(meth)acryloyl-5-aminosalicylic acid, 4-vinyl benzoic acid, 4-(meth)acryloyloxyethoxycarbonyl phthalic acid, 4-(meth)acryloyloxyethoxycarbonyl phthalic acid anhydride, 5-(meth)acryloylaminopentyl carboxylic acid, 11-(meth)acryloyloxy-1,1-undecane dicarboxylic acid, and acid chlorides of these.

Examples of the sulfonic acid group-containing polymerizable monomer include p-styrenesulfonic acid.

In view of excellence of adhesion at the interface, preferred among these compounds are phosphoric acid group-containing polymerizable monomers or phosphonic acid group-containing polymerizable monomers, particularly 10-(meth)acryloyloxydecyl dihydrogen phosphate, 2-(meth)acryloyloxyethylphenyl phosphoric acid, and 2-(meth)acryloyloxyethylphenyl phosphonate. These adhesive monomers (a-4) having an acidic group may be used alone, or two or more thereof may be used.

The content of the adhesive monomer (a-4) having an acidic group in the adhesive composition (F) is not particularly limited, and is preferably 0.1 to 10 mass%, more preferably 1 to 5 mass%, even more preferably 1 to 3 mass% in a total amount of the adhesive composition (F). When the dental curable composition comprises an adhesive monomer (a-4) having an acidic group, the content of the adhesive monomer (a-4) having an acidic group is preferably 1 to 35 parts by mass, more preferably 2 to 30 parts by mass, even more preferably 5 to 25 parts by mass in total 100 parts by mass of polymerizable compound (A).

<Polyfunctional Thiol (B)>

The following describes the polyfunctional thiol (B) having two or more mercapto groups per molecule (hereinafter, also referred to as "polyfunctional thiol (B)") included in the present invention.

The polyfunctional thiol (B) used in the present invention is a compound having two or more mercapto groups per molecule. When combined with the polymerization initiator (D) described below, the polyfunctional thiol (B) can improve the crosslink density by accelerating curing of the polymerizable compound (A), producing an even stronger cured product with high impact resistance. This provides the desired pencil hardness to the cured product. The cured product of the dental curable composition also exhibits superior abrasion resistance due in part to its high impact resistance. The cured product of the dental curable composition also excels in abrasion resistance for the opposing teeth, in addition to possessing excellent abrasion resistance itself. Additionally, because of accelerated curing, the polymerization initiator (D) can be used in reduced amounts, making it possible to reduce shrinkage during cure. Moreover, the mercapto group forms a hydrogen bond with the OH group on the ceramic surface representing the base material, making it possible to enhance the adhesion between the resin layer and the base material, and increase the refractive index by the presence of sulfur atoms in the molecule. This enables a greater reduction of shade changes when the base material is coated with the dental curable composition.

The polyfunctional thiol (B) can also provide even superior gloss retention when combined with a polysiloxane (C) (preferably, polysilsesquioxane (C-1), more preferably a polysilsesquioxane composition that is liquid at 25°C).

The polyfunctional thiol (B) is not particularly limited, as long as it is a compound having two or more mercapto groups per molecule. Specific examples include compounds having two mercapto groups per molecule, compounds having three mercapto groups per molecule, compounds having four mercapto groups per molecule, and compounds having six mercapto groups per molecule. In view of excellence of curability improving effect and shorter curing time, preferred are polyfunctional thiols having 2 to 4 mercapto groups per molecule. In view of the effect to more greatly reduce shade changes in coating the base material, and providing even superior gloss retention, more preferred are polyfunctional thiols having three mercapto groups per molecule, and polyfunctional thiols having four mercapto groups per molecule.

Examples of compounds having two mercapto groups per molecule include:

compounds having two primary mercapto groups per molecule, such as butanediol bis(2-mercaptoacetate), hexanediol bis(2-mercaptoacetate), ethanediol bis(2-mercaptoacetate), 2,2'-(ethylenedithio)diethanethiol, ethylene glycol bis(3-mercapto-2-methylpropionate), propylene glycol bis(3-mercapto-2-methylpropionate), diethylene glycol bis(3-mercapto-2-methylpropionate), butanediol bis(3-mercapto-2-methylpropionate), octanediol bis(3-mercapto-2-methylpropionate), and bis(3-mercapto-2-methylpropyl)phthalate;

compounds having two secondary mercapto groups per molecule, such as 1,4-bis(3-mercaptobutyryloxy)butane, bis(1-mercaptoethyl)phthalate, bis(2-mercaptopropyl)phthalate, bis(3-mercaptobutyl)phthalate, ethylene glycol bis(3-mercaptobutyrate), propylene glycol bis(3-mercaptobutyrate), diethylene glycol bis(3-mercaptobutyrate), butanediol bis(3-mercaptobutyrate), octanediol bis(3-mercaptobutyrate), ethylene glycol bis(2-mercaptopropionate), propylene glycol bis(2-mercaptopropionate), diethylene glycol bis(2-mercaptopropionate), butanediol bis(2-mercaptopropionate), octanediol bis(2-mercaptopropionate), ethylene glycol bis(4-mercaptovalerate), propylene glycol bis(4-mercaptoisovalerate), diethylene glycol bis(4-mercaptovalerate), butanediol bis(4-mercaptovalerate), octanediol bis(4-mercaptovalerate), ethylene glycol bis(3-mercaptovalerate), propylene glycol bis(3-mercaptovalerate), diethylene glycol bis(3-mercaptovalerate), butanediol bis(3-mercaptovalerate), and octanediol bis(3-mercaptovalerate); and

compounds having two tertiary mercapto groups per molecule, such as ethylene glycol bis(2-mercaptoisobutyrate), propylene glycol bis(2-mercaptoisobutyrate), diethylene glycol bis(2-mercaptoisobutyrate), butanediol bis(2-mercaptoisobutyrate), and octanediol bis(2-mercaptoisobutyrate).

In this specification, "primary mercapto group" means a mercapto group attached to a primary carbon atom, "secondary mercapto group" means a mercapto group attached to a secondary carbon atom, and "tertiary mercapto group" means a mercapto group attached to a tertiary carbon atom.

Examples of compounds having three mercapto groups per molecule include:

compounds having three primary mercapto groups per molecule, such as trimethylolpropane tris(2-mercaptoacetate), trimethylolpropane tris(3-mercaptopropionate), trimethylolpropane tris(3-mercapto-2-methylpropionate), and 3-(3-mercapto-propoxy)-2,2-bis-(3-mercapto-propoxymethyl)-propan-1-ol;

compounds having three secondary mercapto groups per molecule, such as trimethylolpropane tris(3-mercaptobutyrate), trimethylolpropane tris(2-mercaptopropionate), trimethylolpropane tris(4-mercaptovalerate), trimethylolpropane tris(3-mercaptovalerate), and 1,3,5-tris(3-mercaptobutyloxyethyl)-1,3,5-triazine-2,4,6(1H,3H,5H)-trione; and

compounds having three tertiary mercapto groups per molecule, such as trimethylolpropane tris(2-mercaptoisobutyrate).

Examples of compounds having four mercapto groups per molecule include:

compounds having four primary mercapto groups per molecule, such as pentaerythritol tetrakis(2-mercaptoacetate), pentaerythritol tetrakis(3-mercaptopropionate), and pentaerythritol tetrakis(3-mercaptobutyrate);

compounds having four secondary mercapto groups per molecule, such as dipentaerythritol hexakis(3-mercaptobutyrate), pentaerythritol tetrakis(2-mercaptopropionate), pentaerythritol tetrakis(3-mercapto-2-propionate), pentaerythritol tetrakis(4-mercaptovalerate), and pentaerythritol tetrakis(3-mercaptovalerate); and

compounds having four tertiary mercapto groups per molecule, such as pentaerythritol tetrakis(2-mercaptoisobutyrate).

Examples of compounds having six mercapto groups per molecule include:

compounds having six primary mercapto groups per molecule, such as dipentaerythritol hexakis(3-mercapto-2-methylpropionate);

compounds having six secondary mercapto groups per molecule, such as dipentaerythritol hexakis(3-mercaptobutyrate), dipentaerythritol hexakis(2-mercaptopropionate), dipentaerythritol hexakis(4-mercaptovalerate), and dipentaerythritol hexakis(3-mercaptovalerate); and

compounds having six tertiary mercapto groups per molecule, such as dipentaerythritol hexakis(2-mercaptoisobutyrate).

In view of suppressing the unpleasant odor of the curable composition, and providing improved storage stability, preferred among these polyfunctional thiols (B) are compounds having no primary mercapto group, and in which the number of secondary mercapto groups and the number of tertiary mercapto groups are two or more in total. For example, preferred are compounds having two secondary mercapto groups per molecule, compounds having two tertiary mercapto groups per molecule, compounds having three secondary mercapto groups per molecule, compounds having three tertiary mercapto groups per molecule, compounds having four secondary mercapto groups per molecule, compounds having four tertiary mercapto groups per molecule, compounds having six secondary mercapto groups per molecule, and compounds having six tertiary mercapto groups per molecule, more preferably compounds having two secondary mercapto groups per molecule, compounds having two tertiary mercapto groups per molecule, compounds having three secondary mercapto groups per molecule, compounds having three tertiary mercapto groups per molecule, compounds having four secondary mercapto groups per molecule, and compounds having four tertiary mercapto groups per molecule, even more preferably compounds having three secondary mercapto groups per molecule, compounds having three tertiary mercapto groups per molecule, compounds having four secondary mercapto groups per molecule, and compounds having four tertiary mercapto groups per molecule.

Particularly preferred as polyfunctional thiol (B) are pentaerythritol tetrakis(3-mercaptobutyrate), and 1,3,5-tris(3-mercaptobutyloxyethyl)-1,3,5-triazine-2,4,6(1H,3H,5H)-trione.

The polyfunctional thiol (B) may be used alone, or two or more thereof may be used in combination. The molecular weight of polyfunctional thiol (B) is not particularly limited. However, in view of improving the curability of the curable composition, the polyfunctional thiol (B) has a molecular weight of preferably 200 to 1,000.

The polyfunctional thiol (B) is also readily available as a commercially available product. Examples of such commercially available products of polyfunctional thiols having two or more mercapto groups per molecule include 1,4-bis(3-mercaptobutyryloxy)butane (KarenzMT® BD1 manufactured by Showa Denko K.K. under this trade name), pentaerythritol tetrakis(3-mercaptobutyrate) (KarenzMT® PE1 manufactured by Showa Denko K.K. under this trade name), 1,3,5-tris(3-mercaptobutyryloxyethyl)-1,3,5-triazine-2,4,6(1H,3H,5H)-trione (KarenzMT® NR1 manufactured by Showa Denko K.K. under this trade name), and trimethylolpropane tris(3-mercaptobutyrate) (TPMB manufactured by Showa Denko K.K. under this trade name).

In view of the gloss of the cured product, the polyfunctional thiol (B) has a refractive index of preferably 1.40 or greater, more preferably 1.45 or greater, even more preferably 1.50 or greater. The refractive index of polyfunctional thiol (B) can be measured with an Abbe refractometer. The refractive index can be measured according to JIS K 0062:1992 with some modification, specifically, by a liquid immersion method at 23°C using an Abbe refractometer, with the sodium D-line serving as the light source. As for the liquid, different liquids with varying refractive indices are prepared by combining two or more liquids whose refractive indices are similar to the presumed refractive index of the sample filler (polyfunctional thiol (B)). The sample is suspended in each liquid in a 23°C atmosphere, and the liquid appearing most transparent as observed by the naked eye is selected. By regarding the refractive index of this liquid as the refractive index of the sample, the liquid's refractive index is measured with an Abbe refractometer. Examples of usable liquids include diiodomethane with sulfur dissolved in it, 1-bromonaphthalene, methyl salicylate, dimethylformamide, and 1-pentanol.

In view of enhancing curability, it is preferable to determine the content of polymerizable compound (A) and polyfunctional thiol (B) based on the number of unsaturated groups in the polymerizable compound (A) and the number of mercapto groups in the polyfunctional thiol (B). That is, the ratio of the number of unsaturated groups in the polymerizable compound (A) with respect to the number of mercapto groups in the polyfunctional thiol (B) ([number of unsaturated groups]/[number of mercapto groups]) ranges preferably from 0.25 to 4. Because the reaction between unsaturated groups and mercapto groups is equivalent, an excess of unsaturated groups compared to mercapto groups may easily lead to the generation of radicals through heat, resulting in degradation of the cured product. Conversely, an excess of mercapto groups compared to unsaturated groups may cause the release of polyfunctional thiol (B) due to the decomposition of the cured product from heat or humid heat, resulting in an undesirable odor.

From the same perspective mentioned above, as well as the improvement of adhesive properties to the base material, the content of polyfunctional thiol (B) is preferably 5 to 40 mass%, more preferably 10 to 35 mass%, even more preferably 15 to 30 mass% in a total amount of the dental curable composition. With the content of the polyfunctional thiol (B) in the curable composition falling in these ranges, it is possible to reduce odor before and after the curing of the curable composition, and provide a cured product having enhanced curability and improved adhesive properties to the base material, in addition to improving abrasion resistance, reducing shade changes in the base material, and enhancing gloss retention, among other improvements.

In the present invention, the number of unsaturated groups in polymerizable compound (A) means the total number (in moles) of the unsaturated groups in all compounds belonging to polymerizable compound (A), and the number of mercapto groups in polyfunctional thiol (B) means the total number (in moles) of the mercapto groups in all compounds belonging to polyfunctional thiol (B).

<Polysiloxane (C)>

The following describes the polysiloxane (C) included in the present invention. The polysiloxane (C) is not particularly limited, as long as it is a polymer in which silicon atoms form bonds with each other through oxygen atoms, and at least some of the silicon atoms are linked to organic groups. The polysiloxane (C) improves the adhesive properties to the base material (preferably, a ceramic base material). The polysiloxane (C) may be used alone, or two or more thereof may be used in combination.

A certain preferred embodiment is, for example, a dental curable composition in which the polysiloxane (C) comprises a polysilsesquioxane (C-1).

The following descriptions of preferred embodiments are based on polysilsesquioxane (C-1).

<Polysilsesquioxane (C-1)>

The polysilsesquioxane (C-1) included in the present invention improves the adhesive properties to the base material (preferably, a ceramic base material). When combined with the filler (E) described below, the polysilsesquioxane (C-1) can interact with the filler (E) to improve the affinity for the polymerizable compound (A), increasing the strength of the cured product.

The polysilsesquioxane (C-1) is not particularly limited, as long as it is a linear, branched, or network compound possessing Si-O bonds (siloxane bonds). Examples of the polysiloxane (C) include those with a constituent unit represented by the following general formula (I). In the following general formula (I), n = 1 in the case of polysilsesquioxane (C-1).

$$R_nSiO_{(4-n)/2} \qquad (I),$$

where R represents an organic group, and n represents a number exceeding 0 and less than 4.

Examples of R in the general formula (I) include an alkyl group, a cycloalkyl group, a haloalkyl group, an alkenyl group, an aryl group, and an arylalkyl group.

Examples of the alkyl group include C1 to C10 alkyl groups such as a methyl group, an ethyl group, a propyl group, and a butyl group.

Examples of the cycloalkyl group include C3 to C10 cycloalkyl groups such as a cyclopentyl group, and a cyclohexyl group.

Examples of the haloalkyl group include C1 to C10 halogenated alkyl groups such as a 3-chloropropyl group, and a 3,3,3-trifluoropropyl group.

Examples of the alkenyl group include C2 to C10 alkenyl groups such as a vinyl group, an allyl group, and a butenyl group.

Examples of the aryl group include C6 to C20 aryl groups such as a phenyl group, a tolyl group, and a naphthyl group.

Examples of the arylalkyl group include C1 to C4 alkyl groups with C6 to C12 aryl groups, such as a benzyl group, and a phenethyl group.

The organic group represented by R is preferably a methyl group, a phenyl group, an alkyl group, an alkenyl group (such as a vinyl group), or a fluoro C1 to C6 alkyl group.

Examples of the polysilsesquioxane (C-1) include polydialkylsiloxanes such as polydimethylsiloxane; polyalkylalkenylsiloxanes such as polymethylvinylsiloxane; polyalkylarylsiloxanes such as polymethylphenylsiloxane; polydiarylsiloxanes such as polydiphenylsiloxane; and copolymers with a constituent polyorganosiloxane unit, such as a dimethylsiloxane-methylvinylsiloxane copolymer, a dimethylsiloxane-methylphenylsiloxane copolymer, a dimethylsiloxane-methyl(3,3,3-trifluoropropyl)siloxane copolymer, and a dimethylsiloxane-methylvinylsiloxane-methylphenylsiloxane copolymer. The polysilsesquioxane (C-1) may be used alone, or two or more thereof may be used in combination.

The polysilsesquioxane (C-1) may use a composition prepared by heat treatment of an organosilicon compound such as a silane coupling agent. An example is a composition prepared by dissolving a silane coupling agent in a solvent, heating the solution at 70°C for 3 hours to obtain a sol solution, and removing the solvent and other components at 70°C by distillation in a vacuum. The polysilsesquioxane (C-1) is preferably one that is liquid at 25°C. The polysilsesquioxane (C-1) obtained by such a method may be used in the form of a composition containing unreacted organosilicon compounds. In view of enhancing the curability of the dental curable composition, the polysilsesquioxane (C-1) may be used in the form of a composition containing unreactants, using organosilicon compounds having unsaturated groups (described later). By using a polysilsesquioxane (polysilsesquioxane composition) that is liquid at 25°C, it is easier to provide a uniform coating, improving the operability in building up the dental curable composition on the base material. By being used with polyfunctional thiol (B), a polysilsesquioxane composition that is liquid at 25°C can also reduce shade changes in coating the base material, providing even superior gloss retention.

Examples of the organosilicon compounds used include:

organosilicon compounds having a phenyl group, such as phenyltriethoxysilane, trimethoxy(phenylethyl)silane (a mixture of 1-phenylethyl and 2-phenylethyl), (1E,4E)-1-(4-hydroxy-3,5-dimethoxyphenyl)-5-(3,4,5-trimethoxyphenyl)-1,4-pentadien-3-one, trimethoxy(4-vinylphenyl)silane, trimethoxy(4-methoxyphenyl)silane, phenyltrimethoxysilane, and benzyltriethoxysilane;

organosilicon compounds having an alkyl group, such as methyltriethoxysilane, dodecyltrimethoxysilane, octadecyltriethoxysilane, n-octyltriethoxysilane, cyclohexyldimethoxymethylsilane, cyclohexyltrimethoxysilane, hexyltrimethoxysilane, ethyltrimethoxysilane, decyltrimethoxysilane, hexadecyltrimethoxysilane, propyltrimethoxysilane, cyclopentyltrimethoxysilane, butyltriethoxysilane, isooctyltriethoxysilane, and isobutyltriethoxysilane; and organosilicon compounds having an unsaturated group, such as trimethoxy(4-vinylphenyl)silane, vinyltrimethoxysilane, 3-(trimethoxysilyl)propyl acrylate, 3-(triethoxysilyl)propyl methacrylate, allyltriethoxysilane, and allyltrimethoxysilane.

The organosilicon compounds may be used alone, or two or more thereof may be used as a mixture. In view of improving the adhesive properties to ceramic base materials, and enhancing the affinity for the polymerizable compound (A) when combined with the filler (E) described below, preferred are organosilicon compounds having a phenyl group, organosilicon compounds having an unsaturated group, and mixtures of these, more preferably phenyltriethoxysilane, 3-(trimethoxysilyl)propyl acrylate, and mixtures of these.

In view of improving the adhesive properties to ceramic base materials, and enhancing the affinity for the polymerizable compound (A) when combined with the filler (E) described below, the content of polysiloxane (C) (preferably, polysilsesquioxane (C-1)) is preferably 0.1 mass% or more, more preferably 0.2 mass% or more, even more preferably 0.5 mass% or more in a total amount of the dental curable composition. In view of the hardness of the cured product, the content of polysiloxane (C) (preferably, polysilsesquioxane (C-1)) is preferably 10 mass% or less, more preferably 8 mass% or less, even more preferably 5 mass% or less. The upper limits and lower limits of these ranges may be freely combined. The content of polysiloxane (C) is, for example, preferably 0.1 to 10 mass%, more preferably 0.2 to 8 mass%, even more preferably 0.5 to 5 mass%.

<Polymerization initiator (D)>

The following describes a polymerization initiator (D) included in the present invention. The polymerization initiator (D) may be selected from polymerization initiators that are commonly available, preferably polymerization initiators used in dentistry in particular. Notably, it is possible to use a photopolymerization initiator (D-1) or a chemical polymerization initiator (D-2). These may be used alone, or two or more thereof may be used in appropriate combinations. Preferred in view of surface curability is photopolymerization initiator (D-1).

Examples of the photopolymerization initiator (D-1) include $\alpha$-diketones, ketals, thioxanthones, acylphosphine oxides, and $\alpha$-aminoacetophenones.

Examples of the $\alpha$-diketones include dl-camphorquinone, benzyl, and 2,3-pentanedione.

Examples of the ketals include benzyl dimethyl ketal, and benzyl diethyl ketal.

Examples of the thioxanthones include 2-chlorothioxanthone, and 2,4-diethylthioxanthone.

Examples of the acylphosphine oxides include

2,4,6-trimethylbenzoyldiphenylphosphine oxide, bis(2,4,6-trimethylbenzoyl)phenylphosphine oxide, bis(benzoyl)phenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl di(2,6-dimethylphenyl)phosphonate, 2,4,6-trimethylbenzoylethoxyphenylphosphine oxide, and water-soluble acylphosphine oxide compounds disclosed in JP H03-57916 B.

Examples of the $\alpha$-aminoacetophenones include 2-benzyl-dimethylamino-1 -(4-morpholinophenyl)-1 -butanone, 2-benzyl-diethylamino-1-(4-morpholinophenyl)-1-butanone, 2-benzyl-dimethylamino-1-(4-morpholinophenyl)-1-propanone, 2-benzyl-diethylamino-1-(4-morpholinophenyl)-1-propanone, 2-benzyl-dimethylamino-1-(4-morpholinophenyl)-1-pentanone, and 2-benzyl-diethylamino-1-(4-morpholinophenyl)-1-pentanone.

The photopolymerization initiator (D-1) may be used alone, or two or more thereof may be used in combination. The content of photopolymerization initiator (D-1) is preferably 0.01 to 15 parts by mass, more preferably 0.05 to 12 parts by mass, even more preferably 0.1 to 10 parts by mass in total 100 parts by mass of the polymerizable compound (A).

The photopolymerization initiator (D-1) may be used by itself, or, in order to accelerate light curing, the photopolymerization initiator (D-1) may be used with a polymerization accelerator such as a tertiary amine, an aldehyde, or a compound having a thiol group.

Examples of the tertiary amine include aliphatic tertiary amines such as 2-(dimethylamino)ethyl (meth)acrylate, N,N-bis[(meth)acryloyloxyethyl]-N-methylamine, N-methyldiethanolamine di(meth)acrylate, N-ethyldiethanolamine di(meth)acrylate, triethanolamine mono(meth)acrylate, triethanolamine di(meth)acrylate, triethanolamine tri(meth)acrylate, triethanolamine, trimethylamine, triethylamine, tributylamine, N-methyldiethanolamine, N-ethyldiethanolamine, and N-n-butyldiethanolamine; and aromatic tertiary amines such as ethyl 4-(N,N-dimethylamino)benzoate, butyl 4-(N,N-dimethylamino)benzoate, butoxyethyl 4-(N,N-dimethylamino)benzoate, N-methyldiethanolamine, 4-(N,N-dimethylamino)benzophenone, and N,N-di(2-hydroxyethyl)-p-toluidine.

Examples of the aldehyde include dimethylaminobenzaldehyde, and terephthalaldehyde.

Examples of the compound having a thiol group include compounds having one mercapto group per molecule, such as 2-mercaptobenzoxazole, decanethiol, 3-mercaptopropyltrimethoxysilane, and thiobenzoic acid.

The polymerization accelerator may be used alone, or two or more thereof may optionally be used in combination. The content of the polymerization accelerator is preferably 0.01 to 15 parts by mass, more preferably 0.05 to 12 parts by mass, even more preferably 0.1 to 10 parts by mass in total 100 parts by mass of the polymerizable compound (A).

The chemical polymerization initiator (D-2) is preferably a redox polymerization initiator composed of an oxidizing agent and a reducing agent. When using a redox polymerization initiator, the polymerizable compound (A) must be packed in at least two to separate the oxidizing agent and the reducing agent.

Examples of the oxidizing agent of the redox polymerization initiator include organic peroxides such as diacyl peroxides, peroxyesters, dialkyl peroxides, peroxyketals, ketone peroxides, and hydroperoxides.

Specific examples of the diacyl peroxides include benzoyl peroxide, 2,4-dichlorobenzoyl peroxide, and m-toluoyl peroxide. Specific examples of the peroxyesters include t-butyl peroxybenzoate, bis-t-butyl peroxyisophthalate, 2,5-dimethyl-2,5-bis(benzoylperoxy)hexane, t-butyl peroxy-2-ethylhexanoate, and t-butyl peroxyisopropyl carbonate. Specific examples of the dialkyl peroxides include dicumyl peroxide, di-t-butyl peroxide, and lauroyl peroxide. Specific examples of the peroxyketals include 1,1-bis(t-butylperoxy)-3,3,5-trimethylcyclohexane. Specific examples of the ketone peroxides include methyl ethyl ketone peroxide, cyclohexanone peroxide, and methyl acetoacetate peroxide. Specific examples of the hydroperoxides include t-butyl hydroperoxide, cumene hydroperoxide, and p-diisopropyl benzene peroxide.

Examples of the reducing agent of the redox polymerization initiator include aromatic tertiary amines, aliphatic tertiary amines, and sulfinic acid and its salts.

Examples of the aromatic tertiary amines include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-di(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N, N-bis(2-hydroxyethyl)-3,5-di-isopropylaniline, N,N-bis(2-hydroxyethyl)-3,5-dibutylaniline, ethyl 4-(N,N-dimethylamino)benzoate, n-butoxyethyl 4-(N,N-dimethylamino)benzoate, (2-methacryloyloxy)ethyl 4-(N,N-dimethylamino)benzoate, and 4-(N,N-dimethylamino)benzophenone.

Examples of the aliphatic tertiary amines include trimethylamine, triethylamine, N-methyldiethanolamine, N-ethyldiethanolamine, N-n-butyldiethanolamine, N-lauryldiethanolamine, triethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, and triethanolamine trimethacrylate.

Examples of sulfinic acid and its salts include benzenesulfinic acid, sodium benzenesulfinate, potassium benzenesulfinate, calcium benzenesulfinate, lithium benzenesulfinate, p-toluenesulfinic acid, sodium p-toluenesulfinate, potassium p-toluenesulfinate, calcium p-toluenesulfinate, lithium p-toluenesulfinate, 2,4,6-trimethylbenzenesulfinic acid, sodium 2,4,6-trimethylbenzenesulfinate, potassium 2,4,6-trimethylbenzenesulfinate, calcium 2,4,6-trimethylbenzenesulfinate, lithium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, and calcium 2,4,6-triisopropylbenzenesulfinate.

The oxidizing agent and the reducing agent each may be used alone, or two or more thereof may optionally be used in combination. The content of the oxidizing agent and the content of the reducing agent are both preferably 0.01 to 15 parts by mass, more preferably 0.05 to 12 parts by mass, even more preferably 0.1 to 10 parts by mass relative to total 100 parts by mass of the polymerizable compound (A).

<Filler (E)>

A dental curable composition of the present invention may comprise a filler (E), in order to enhance mechanical strength and abrasion resistance, and to adjust properties such as coatability and flowability during application. The filler (E) may be an inorganic filler, an organic filler, or inorganic/organic composite filler.

Examples of the inorganic filler include minerals containing silica as the base material, such as silica, kaolin, clay, *ummo*, and mica; and ceramics or glass containing compounds such as $Al_2O_3$, $B_2O_3$, $TiO_2$, $ZrO_2$, $BaO$, $La_2O_3$, $SrO_2$, $CaO$, or $P_2O_5$, in addition to silica serving as the base material. Specific examples of such glass include lanthanum glass, barium glass, strontium glass, soda glass, lithium borosilicate glass, aluminosilicate glass, barium boroaluminosilicate glass, strontium boroaluminosilicate glass, zinc glass, fluoroaluminosilicate glass, borosilicate glass, calcium fluoroaluminosilicate glass, strontium fluoroaluminosilicate glass, barium fluoroaluminosilicate glass, strontium calcium fluoroaluminosilicate glass, and bioglass. Other examples of the inorganic filler include crystal quartz, hydroxyapatite, alumina, titania, yttrium oxide, zirconia, calcium phosphate, barium sulfate, aluminum hydroxide, sodium fluoride, potassium fluoride, sodium monofluorophosphate, lithium fluoride, calcium fluoride, ytterbium fluoride, and yttrium fluoride. Preferred among these are silica, alumina, and titania, more preferably silica.

In view of enhancing the strength of the cured product, the inorganic filler has a particle diameter of preferably 100 nm or less, more preferably 95 nm or less, even more preferably 90 nm or less, particularly preferably 80 nm or less in terms of an average primary particle diameter. In view of the viscosity of the composition, the average primary particle diameter is preferably 5 nm or more, more preferably 15 nm or more, even more preferably 20 nm or more, particularly preferably 25 nm or more. The average primary particle diameter may fall in any combinations of these ranges. For example, the average primary particle diameter is preferably 5 to 100 nm, preferably 15 to 95 nm, more preferably 20 to 90 nm, even more preferably 25 to 80 nm.

A certain embodiment is, for example, a dental curable composition in which the inorganic filler (preferably, hydrophobic silica (E-1)) has an average primary particle diameter of 15 to 100 nm.

In view of enhancing the affinity for the polymerizable compound (A) and/or polyfunctional thiol (B), and improving the antifouling properties, it is preferable that the inorganic filler comprise a hydrophobic inorganic filler, more preferably a hydrophobic silica (E-1).

The hydrophobic inorganic filler has a methanol hydrophobicity of preferably 15% or more, more preferably 20% or more, even more preferably 25% or more.

The methanol hydrophobicity is measured as follows. Specifically, 0.1 g of a sample is weighed and placed in a 200 mL beaker, followed by addition of 50 mL of ion-exchange water, and stirring with a magnetic stirrer. Subsequently, methanol is added dropwise using a burette, at a rate of about 2 mL every 10 seconds, until the sample floating on the liquid surface completely disappears, indicating the end point. The methanol hydrophobicity is then calculated using the following formula.

$$\text{Methanol hydrophobicity (\%)} = [\text{volume of titrant} / (\text{volume of titrant} + 50)] \times 100$$

In view of improving affinity by enhancing its interaction with polysiloxane (C), the inorganic filler is preferably one that has been surface-treated with a surface treatment agent. This not only helps prevent aggregation of inorganic fillers, leading to a reduction in the viscosity of the composition, but also improves the strength and abrasion resistance of the cured product. The surface treatment agent may be, for example, a silane coupling agent. Examples of the silane coupling agent include, but are not particularly limited to, methyltrimethoxysilane, dimethyldimethoxysilane, phenyltrimethoxysilane, diphenyldimethoxysilane, methyltriethoxysilane, dimethyldiethoxysilane, phenyltriethoxysilane, diphenyldiethoxysilane, isobutyltrimethoxysilane, vinyltrimethoxysilane, vinyltriethoxysilane, vinyltris(2-methoxyethoxy)silane, 3,3,3-trifluoropropyltrimethoxysilane, methyl-3,3,3-trifluoropropyldimethoxysilane, 2-(3,4-epoxycyclohexyl)ethyltrimethoxysilane, 3-glycidoxypropyltrimethoxysilane, 3-glycidoxypropylmethyldiethoxysilane, 3-glycidoxypropyltriethoxysilane, 3-methacryloyloxypropylmethyldimethoxysilane, γ-methacryloyloxypropylmethyldiethoxysilane, N-β(aminoethyl)γ-aminopropylmethyldimethoxysilane, N-β(aminoethyl)γ-aminopropyltrimethoxysilane, N-β(aminoethyl)γ-aminopropyltriethoxysilane, 3-aminopropyltrimethoxysilane, 3-aminopropyltriethoxysilane, N-phenyl-γ-aminopropyltrimethoxysilane, 3-mercaptopropyltrimethoxysilane, trimethylsilanol, methyltrichlorosilane, methyldichlorosilane, dimethyldichlorosilane, trimethylchlorosilane, phenyltrichlorosilane, diphenyldichlorosilane, vinyltrichlorosilane, trimethylbromosilane, diethylsilane, vinyltriacetoxysilane, ω-(meth)acryloyloxyalkyltrimethoxysilane (3 to 12 carbon atoms between the (meth)acryloyloxy group and the silicon atom, for example, such as γ-methacryloyloxypropyltrimethoxysilane), ω-(meth)acryloyloxyalkyltriethoxysilane (3 to 12 carbon atoms between the (meth)acryloyloxy group and the silicon atom, for example, such as γ-methacryloyloxypropyltriethoxysilane).

The particle shape is not particularly limited, and the surface treatment agent may be used as a powder of irregularly shaped particles or spherical particles.

The inorganic filler may be a commercially available product. Examples include:

Aerosil® OX50, Aerosil® 50, Aerosil® 130, Aerosil® 200, Aerosil® 380, Aerosil® MOX80, Aerosil® R972, AEROSIL® R7200, Aerosil® RY50, Aerosil® NAX50, Aerosil® NX90G, Aeroxide® AluC, Aeroxide® TiO2 P25, VP Zirconium Oxide 3-YSZ, and VP Zirconium Oxide 3-YSZ PH (manufactured by EVONIK INDUSTRIES, distributed by Nippon Aerosil Co., Ltd.);
QSG-30 and QSG-80 manufactured by Shin-Etsu Chemical Co., Ltd.;
MSP-001, MSP-005, MSP-009, MSP-011, MSP-012, MSP-013, MSP-015, and MSP-016 manufactured by TAYCA Co., Ltd.; and
YA050C-SP3, YA050C, YA010C, and YC100C manufactured by Admatechs.

Preferred among these are AEROSIL® R7200 (fine silica particles, average primary particle diameter: 12 nm, hydrophobic fumed silica, surface-treated with a methacryloyloxysilyl group-containing silane compound), Aerosil® NAX50 (fine silica particles, average primary particle diameter: 30 nm, methanol hydrophobicity: 28%, surface-treated with hexamethyldisilazane), QSG-30 (fine spherical silica particles, average primary particle diameter: 30 nm, methanol hydrophobicity: 67%, surface-treated with methyltrimethoxysilane and hexamethyldisilazane), MSP-011 (fine silica particles, average primary particle diameter: 30 nm, methanol hydrophobicity: 41%, surface-treated with methyltrimethoxysilane and hexamethyldisilazane), and YA050C-SP3 (fine spherical silica particles, average primary particle diameter: 50 nm, methanol hydrophobicity: 47%, surface-treated with phenylmethoxysilane).

The average primary particle diameter can be determined by a laser diffraction scattering method or by electron microscopy of particles. Specifically, a laser diffraction scattering method is more convenient for the measurement of particles that are 0.1 μm or larger, whereas electron microscopy is a more convenient method of particle diameter measurement for ultrafine particles of less than 0.1 μm. Here, 0.1 μm is a measured value by a laser diffraction scattering method. As an example of a laser diffraction scattering method, the particle size may be measured by volume using a laser diffraction particle size distribution analyzer (SALD-2300, Shimadzu Corporation) with a 0.2% sodium hexametaphosphate aqueous solution used as dispersion medium. A scanning electron microscope (e.g., SU3800 or S-4000 manufactured by Hitachi High-Technologies Corporation) may be used for electron microscopy. In electron microscopy, the particle size can be measured by taking an electron micrograph of particles, and the size of particles (at least 200 particles) observed in a unit field of the captured image may be measured using image-analyzing particle-size-distribution measurement software (Mac-View, Mountech Co., Ltd.). Here, the particle diameter is determined as an arithmetic mean value of the maximum and minimum lengths of particles, and the average primary particle diameter is calculated from the number of particles and the particle diameter.

Examples of the organic filler include polymethyl methacrylate, polyethyl methacrylate, a copolymer of polymethyl methacrylate and polyethyl methacrylate, a polymer of polyfunctional methacrylate, an ethylene-vinyl acetate copolymer, an acrylonitrile-butadiene-styrene copolymer, polyamides, polystyrene, polyvinyl chloride, chloroprene rubber, nitrile rubber, and styrene-butadiene rubber.

Examples of the inorganic/organic composite filler include those comprising an organic filler, and an inorganic filler dispersed in the organic filler, and those comprising an inorganic filler with various types of polymerizable monomers (A-2) coating the surface of the inorganic filler.

The filler (E) may be used alone, or two or more thereof may optionally be used in combination. In view of providing sufficient impact resistance, the content of filler (E) is preferably 20 mass% or less, more preferably 10 mass% or less, even more preferably 5 mass% or less in a total amount of the dental curable composition. In view of providing sufficient levels of abrasion resistance and antifouling properties, the content of filler (E) is preferably 0.1 mass% or more, more preferably 0.5 mass% or more, even more preferably 1.0 mass% or more. The content of filler (E) may fall in any combinations of these ranges. For example, the content of filler (E) is preferably 0.1 to 20 mass%, more preferably 0.5 to 10 mass%, even more preferably 1.0 to 5.0 mass%.

Optionally, the dental curable compositions used in the present invention may appropriately comprise additives such as solvents (e.g., organic solvents), colorants (pigments, fluorescent pigments), ultraviolet absorbers, or antioxidants to such an extent that the addition of such additives does not hinder the effectiveness of the present invention.

In view of coatability on the base material, a dental curable composition of the present invention has a viscosity of preferably 10 to 1,000 mPa s, more preferably 20 to 800 mPa s, even more preferably 40 to 600 mPas. With these viscosity ranges, the curable composition can easily be coated over the desired area without causing runniness. The viscosity can be measured with, for example, a B-type viscometer (Brookfield viscometer) at ordinary temperature at 20 rpm.

In view of abrasion resistance and impact resistance, a cured film (cured product) of a dental curable composition of the present invention has a pencil hardness of preferably F or higher, more preferably H or higher, even more preferably 2H or higher.

The pencil hardness of a cured film can be measured following JIS K 5600-5-4:1999, for example.

An embodiment in another aspect of the present invention is, for example, a dental curable composition (X) that comprises a polymerizable compound (A), a polysilsesquioxane (C-1), and a polymerization initiator (D), and in which the polysilsesquioxane (C-1) is liquid at 25°C. The components of the dental curable composition (X) are as described above.

The dental curable composition (X) does not comprise a polyfunctional thiol (B). However, the dental curable composition (X) may comprise optional components (for example, a filler (E), a polymerization accelerator, and additives), as does the dental curable composition comprising a polymerizable compound (A), a polyfunctional thiol (B) having two or more mercapto groups per molecule, a polysilsesquioxane (C-1), and a polymerization initiator (D).

The dental curable composition (X) also share similar specific properties such as viscosity and pencil hardness.

By comprising a polysilsesquioxane (a polysilsesquioxane composition) that is liquid at 25°C, the dental curable composition (X) can more easily achieve a uniform coating, enhancing the operability in building up the dental curable composition on the base material.

<Base Material>

Preferably, a dental curable composition of the present invention is used for applications as a coating agent for coating a base material. The following describes a base material coated in the present invention. The base material used may be selected from dental prosthesis materials that are commonly available, and comprises preferably at least one selected from the group consisting of zirconia, alumina, fused quartz, lithium silicate glass, an acrylic resin, and a composite material containing a polymer resin and an inorganic filler, more preferably zirconia. Here, "zirconia" comprises zirconia as the main component, and includes zirconia sintered bodies that result from sintering of zirconia.

Zirconia sintered bodies are primarily sintered crystal grains of zirconia. Accordingly, this specification is described through the case of zirconia that has not been sintered. However, in these descriptions, the term "zirconia" is interchangeable with "zirconia sintered body", and *vise versa*, provided that it does not pose any issue and unless otherwise specifically stated. For example, a zirconia sintered body may comprise zirconia, and a stabilizing agent capable of preventing a phase transformation of zirconia (hereinafter, also referred to simply as "stabilizing agent"). That is, a base material before sintering may comprise zirconia, and a stabilizing agent capable of preventing a phase transformation of zirconia.

Examples of the stabilizing agent include oxides such as calcium oxide (CaO), magnesium oxide (MgO), yttrium oxide ($Y_2O_3$), cerium oxide ($CeO_2$), scandium oxide ($Sc_2O_3$), niobium oxide ($Nb_2O_5$), lanthanum oxide ($La_2O_3$), erbium oxide ($Er_2O_3$), praseodymium oxide ($Pr_2O_3$, PreOn), samarium oxide ($Sm_2O_3$), europium oxide ($Eu_2O_3$), thulium oxide ($Tm_2O_3$), gallium oxide ($Ga_2O_3$), indium oxide ($In_2O_3$), and ytterbium oxide ($Yb_2O_3$). Preferred is yttria. The stabilizing agent may be incorporated alone, or two or more thereof may be incorporated in combination.

In the present invention, the content of the stabilizing agent in zirconia can be measured by, for example, inductively coupled plasma (ICP) emission spectral analysis or X-ray fluorescence analysis (XRF).

In view of the strength and translucency of the sintered body, the yttria content of when the stabilizing agent contains yttria is preferably 3.0 to 7.5 mol%, more preferably 3.5 to 7.0 mol%, even more preferably 4.0 to 6.5 mol% relative to the total mole of zirconia and stabilizing agent. The sintered body can increase its translucency with a yttria content of 3.0 mol% or more, whereas a decrease in the strength of the sintered body can be reduced with a yttria content of 7.5 mol% or less.

When the zirconia contains calcium oxide, the content of calcium oxide is preferably 1 mol% or less, more preferably 0.3 mol% or less in total 100 mol% of zirconia and stabilizing agent.

When the zirconia contains magnesium oxide, the content of magnesium oxide is preferably 1 mol% or less, more preferably 0.3 mol% or less in total 100 mol% of zirconia and stabilizing agent.

When the zirconia contains cerium oxide, the content of cerium oxide is preferably 1 mol% or less, more preferably 0.3 mol% or less in total 100 mol% of zirconia and stabilizing agent.

Aside from a sintered body obtained by sintering molded zirconia particles under ordinary pressure or under no applied pressure, the zirconia sintered body includes sintered bodies compacted by a high-temperature pressing process such as HIP (Hot Isostatic Pressing).

Preferably, the zirconia sintered body has at least one of partially stabilized zirconia and fully stabilized zirconia as the matrix phase. In the zirconia sintered body, the primary crystalline phase of zirconia is at least one of tetragonal crystal and cubical crystal. The zirconia may comprise both tetragonal crystal and cubical crystal. Preferably, the zirconia sintered body is essentially free of monoclinic crystal. Zirconia that is partially stabilized by adding a stabilizing agent is called partially stabilized zirconia (PSZ), whereas the term fully stabilized zirconia is used to refer to zirconia that is fully stabilized.

The shape and size (dimensions) of the base material can be appropriately selected according to factors such as use, and the oral environment of the patient.

The following describes a dental prosthesis of the present invention. A dental prosthesis of the present invention is a dental prosthesis comprising a base material and a resin layer, and the resin layer comprises a polymerized and cured product of a curable composition comprising a polymerizable compound (A), a polyfunctional thiol (B), a polysiloxane (C), and a polymerization initiator (D). The base material is coated with the resin layer, partly or completely. The resin layer is obtained by applying the dental curable composition onto the base material, followed by polymerization and cure.

A dental prosthesis of the present invention may comprise two or more resin layers of different compositions. Two or more resin layers may be layered on the base material.

The translucency improves when the surface of the base material is coated with the resin layer, compared with the base material alone. When excessively thin, the resin layer fails to sufficiently exhibit the translucency improving effect, whereas the translucency tends to decrease when the resin layer is excessively thick. Accordingly, the thickness of the resin layer on the base material (the total thickness when two or more resin layers of different compositions are present) is preferably 1 to 500 μm, more preferably 7 to 100 μm, even more preferably 7 to 80 μm, most preferably 7 to 50 μm. Preferably, the resin layer thickness is the thickness at the thickest portion of the resin layer.

A method of production of the dental prosthesis is described below.

First, a base material of a predetermined shape and dimensions is prepared. For example, the base material is fabricated into a dental prosthesis for the patient, using a known method. This is followed by application of the dental curable composition to the base material. Optionally, an adhesive composition (F), separately prepared in advance, is applied to the area of base material where the dental curable composition is applied. A brush can be used for application, for example. The area of base material where the dental curable composition is applied may be appropriately selected according to the oral environment of the patient. Preferably, the dental curable composition is applied to an area that will be exposed inside the oral cavity. The dental curable composition may be applied to an area facing the abutment tooth. The dental curable composition is polymerized together with base material after the application of the dental curable composition. For example, polymerization can take place for 90 seconds with an irradiator that emits light of the 500 nm wavelength range. The same process may be repeated when forming a plurality of resin layers as a polymerized and cured product of the dental curable composition. A dental prosthesis of the present invention can then be obtained after optional shape modification and polishing.

In another preferred method, an adhesive composition (F) comprising an adhesive monomer (a-4) having an acidic group may bond the interface between the base material and the dental curable composition. From the perspective of both ease of application and adhesive properties, another preferred method applies the dental curable composition after a primer composition (F) comprising an adhesive monomer (a-4) having an acidic group and a volatile organic solvent (G) is applied to the base material in advance. As an example, the volatile organic solvent (G) contained in the primer composition (F) is preferably one having a boiling point of preferably 150°C or less, more preferably 100°C or less at ordinary pressure. Using a volatile organic solvent having a boiling point exceeding 150°C at ordinary pressure may result in a decrease in the surface curability of the dental curable composition. Examples of the volatile organic solvent include alcohols such as ethanol, methanol, 1-propanol, and isopropyl alcohol; ketones such as acetone, methyl ethyl ketone, and diethyl ketone; ethers such as 1,2-dimethoxyethane, 1,2-diethoxyethane, and tetrahydrofuran; carboxylic acid esters such as methyl formate, ethyl formate, methyl acetate, ethyl acetate, propyl acetate, and butyl acetate; and (meth)acrylic acid esters such as methyl (meth)acrylate, ethyl (meth)acrylate, n-propyl (meth)acrylate, and isopropyl (meth)acrylate. In view of the ability to cure simultaneously with the polymerizable monomers, (meth)acrylic acid esters are preferred. In view of even lower toxicity and lower boiling point, methyl methacrylate is particularly preferred.

The volatile organic solvent (G) may be used alone, or two or more thereof may be used in combination. In view of coatability and operability, the content of volatile organic solvent (G) is preferably 90 to 99.9 mass%, more preferably 95 to 99 mass%, even more preferably 97 to 99 mass% relative to a total amount of primer composition (F).

A dental prosthesis of the present invention can achieve higher translucency than

that achievable with the base material alone. A dental prosthesis of the present invention can also achieve a higher level of glossiness compared to the base material on its own. In this way, a dental prosthesis of the present invention can present an appearance that more closely resemble natural teeth than when the base material is used alone.

The present invention encompasses embodiments combining the foregoing features in various ways within the technical idea of the present invention, provided that the present invention can exhibit its effects.

EXAMPLES

The present invention is described below in greater detail by way of Examples. However, the present invention is in no way limited by the following descriptions, and various changes may be made by a person with ordinary knowledge in the art within the technical idea of the present invention.

[Urethane (meth)acrylate oligomer (A-1)]

UV-1700B: urethane acrylate (UV-1700B manufactured by Mitsubishi Chemical Corporation under this trade name)
UA-306T pentaerythritol triacrylate toluene diisocyanate urethane prepolymer (UA-306T manufactured by Kyoeisha Chemical Co., Ltd. under this trade name)

[Polymerizable monomer (A-2)]

SR454NS: ethoxylated (3)trimethylol propane triacrylate (SR454NS manufactured by Sartomer Company, Exton, PA)
SR9003NS: propoxylated (2)neopentyl glycol diacrylate (SR9003NS manufactured by Sartomer Company, Exton, PA)
DPHA: dipentaerythritol hexaacrylate (DPHA manufactured by Sartomer Company, Exton, PA)
TMPTA: trimethylolpropane trimethacrylate
MMA: methyl methacrylate
D2.6E: 2,2-bis[4-methacryloyloxypolyethoxyphenyl]propane (with an average of 2.6 moles of ethoxy group added)
3G: triethylene glycol dimethacrylate
MDP: 10-methacryloyloxydecyl dihydrogen phosphate

[Polyfunctional thiol (B)]

MT-PE-1: pentaerythritol tetrakis(3-mercaptobutyrate) (KarenzMT® PE1 manufactured by Showa Denko K.K. under this trade name)
3-MPO: 3-(3-mercapto-propoxy)-2,2-bis-(3-mercapto-propoxymethyl)-propan-1-ol

[Monofunctional thiol]

3-MPE: ethyl 3-mercaptopropionate

[Polysiloxane (C)]

A sol solution of a polysilsesquioxane composition was prepared by mixing 5.9 g (24.5 mmol) of phenyltriethoxysilane, 51.1 g (218.1 mmol) of 3-(trimethoxysilyl)propyl acrylate, 23.4 g (1.3 mol) of water, 9.2 g (0.2 mol) of ethanol, and 0.3 g (4.2 mmol) of acetic acid, and heating the mixture at 70°C for 3 hours. Subsequently, the solvent was distilled away by applying heat at 70°C for 10 minutes in a vacuum, yielding a polysilsesquioxane (C-1) that is liquid at 25°C.
KMP-702: polysilsesquioxane particles manufactured by Shin-Etsu Chemical Co., Ltd.

[Polymerization initiator (D)]

BAPO: bis(2,4,6-trimethylbenzoyl)phenylphosphine oxide
Lucirin-TPO: 2,4,6-trimethylbenzoyldiphenylphosphine oxide
CQ: dl-camphorquinone

[Filler (E)]

QSG-30: Fine spherical silica particles manufactured by Shin-Etsu Chemical Co., Ltd. (average primary particle diameter: 30 nm, methanol hydrophobicity: 67%, surface-treated with methyltrimethoxysilane and hexamethyldisilazane)
R7200: AEROSIL® R7200 manufactured by EVONIK INDUSTRIES under this trade name (average primary particle diameter: 12 nm, hydrophobic fumed silica, surface-treated with a methacryloyloxysilyl group-containing silane compound)
Ba-1: A filler surface-treated with 7 parts by mass of γ-methacryloyloxypropyltrimethoxysilane with respect to 100 parts by mass of barium glass (GM27884 NF180 manufactured by Schott; average primary particle diameter: 0.18 μm)
Ba-2: A filler surface-treated with 8 parts by mass of γ-methacryloyloxypropyltrimethoxysilane with respect to 100 parts by mass of barium glass (GM27884 UF0.4 manufactured by Schott; average primary particle diameter: 0.4 μm)

[Other]

DMBE: ethyl 4-(N,N-dimethylamino)benzoate
DMEM: 2-(N,N-dimethylamino)ethyl methacrylate

Examples 1 to 13 and Comparative Examples 1 to 3, and 6 to 7

Dental curable compositions and base materials were prepared for Examples and Comparative Examples as follows, and their properties were evaluated. The results are presented in Table 1.

[Preparation of Dental Curable Composition]

To prepare the compositions, the components shown in Table 1 were mixed at ordinary temperature in the mass ratios shown in Table 1, using a planetary mixer (Mazerustar, manufactured by Kurabo Ltd.).

[Preparation of Zirconia Ceramic Material as Base Material]

To prepare samples corresponding to dental prostheses of the present invention, a dental zirconia (KATANA® Ziroconia STML NW manufactured by Kuraray Noritake Dental Inc. under this trade name; thickness: 14 mm) was milled into a hemispherical shape (12 mm in diameter), a cuboidal shape (2.4 mm in thickness, 36 mm in length, 24 mm in width), or a disc shape (22 mm in diameter, 1.2 mm in thickness), using a dental milling machine DWX-51D (manufactured by Roland DG Corporation).
Subsequently, the shaped product was fired at 1,550°C for 2 hours with a furnace (Noritake KATANA® F-1N manufactured by SK Medical Electronics Co., Ltd. under this trade name), yielding a base material formed of a zirconia sintered body of a hemispherical shape (10 mm in diameter), a cuboidal shape (2 mm in thickness, 30 mm in length, 20 mm in width), or a disc shape (18 mm in diameter, 1 mm in thickness). The hemisphere surface or disc surface was then sandblasted with 50 μm alumina particles under 0.2 MPa pressure, and the base material was ultrasonically washed in acetone, and dried in preparation for coating.

Comparative Example 4

A commercially available product Seeseed® N Color Coat Clear (manufactured by Kuraray Noritake Dental Inc.) containing monomers (polyfunctional acrylate, methyl methacrylate), a surface-treated silica micro filler, and a photopolymerization catalyst was used as a base-material coating material.

Comparative Example 5

A commercially available product Cerabien® ZR FC Paste Stain Clear Glaze (manufactured by Kuraray Noritake Dental Inc.) containing potassium·aluminosilicate glass, an inorganic pigment, glycerin, and 1,3-butanediol was used as a base-material coating material.

[Viscosity Measurement of Dental Curable Composition]

The compositions prepared earlier were measured with a B-type viscometer (Brookfield viscometer) under 25°C, 20 rpm conditions, and mean values were calculated (n = 3).

[Measurement of Pencil Hardness of Polymerized and Cured Dental Curable Composition]

The cured products of the compositions prepared earlier were measured according to JIS K 5600-5-4:1999 (n = 1).

[Evaluation of Operability of Dental Curable Composition]

The compositions prepared earlier were evaluated for the operability of application on the zirconia base materials prepared, using the following criteria (n = 5).

Good: Formation of a uniform thin film is possible by single application
Moderate: streaks or lines occur after single application, but formation of a uniform thin film is possible after multiple applications
Poor: Formation of a uniform coating surface is not possible even after multiple applications

[Evaluation of Abrasion Resistance Simulating Occlusion against Polymerized and Cured Dental Curable Composition]

A Clearfil® Ceramic® Primer Plus (manufactured by Kuraray Noritake Dental Inc.) was applied to the hemispherical zirconia base material prepared earlier. After drying the material by blowing air, the composition prepared earlier was applied, and cured into a specimen under the curing conditions shown in Table 1 (thermal polymerization only in Comparative Example 5). For photoirradiation, Alpha Light® III (manufactured by J. Morita Corp.) was used.

Separately, a bovine enamel prepared in advance was ground at #1000 to grind out a flat surface.

The specimen was set on an abrasion tester (manufactured by NAVIC Co., Ltd.) along with the bovine enamel with the ground flat surface. The hemispherical sample, contacting the secured bovine enamel (initial state), was then rotated 35° under an applied load in room temperature water while maintaining contact. This procedure was completed in 1 second under a 7.8 kgf load. After 1 second, the sample was brought back to the initial state by releasing the load while rotating it 35°. This cycle was repeated 100,000 times.

The width and depth of the wear mark were then measured as a measure of the amount of wear (wear volume, wear diameter, wear depth) in the bovine enamel, using the flat area of bovine enamel that did not contact the sample as a reference (a wear depth of 0 $\mu$m). For the measurement, a surface roughness meter Laser Focus Displacement Gauge LT-8100 (manufactured by Keyence) was used. The result was determined as an index of the abrasion resistance of the dental mill blank for the opposing tooth. Concerning the amount of wear in bovine enamel, the measurement was conducted three times for each of wear volume, wear diameter, and wear depth per sample, and mean values from two samples were calculated.

The preferred wear volume is 0.15 mm$^3$ or less, more preferably 0.12 mm$^3$ or less, even more preferably 0.10 mm$^3$ or less, particularly preferably 0.08 mm$^3$ or less, most preferably 0.06 mm$^3$ or less.

The wear volume may be, for example, 0.02 mm$^3$ or more.

The preferred wear diameter is 2.1 mm or less, more preferably 2.0 mm or less, even more preferably 1.9 mm or less, particularly preferably 1.8 mm or less, most preferably 1.7 mm or less.

The preferred wear depth is 70 $\mu$m or less, more preferably 65 $\mu$m or less, even more preferably 60 $\mu$m or less, particularly preferably 55 $\mu$m or less.

The wear depth may be, for example, 10 $\mu$m or more.

For the measurement of an amount of wear of the polymerized and cured product (coating layer), the wear volume was measured with a digital microscope (VHX-S50 manufactured by Keyence under this trade name).

The amount of wear in the polymerized and cured product was determined by taking mean values, in the same manner as the amount of wear in the bovine enamel.

The preferred wear volume in the coating layer is 0.075 mm$^3$ or less, more preferably 0.07 mm$^3$ or less, even more preferably 0.065 mm$^3$ or less, particularly preferably 0.06 mm$^3$ or less. The wear volume may be, for example, 0.02 mm$^3$ or more.

[Wear Assessment of Polymerized and Cured Dental Curable Composition from Tooth Brushing]

A Clearfil® Ceramic® Primer Plus (manufactured by Kuraray Noritake Dental Inc.) was applied onto the cuboidal zirconia base material prepared earlier. After drying the material by blowing air, the composition prepared earlier was applied, and cured into a specimen under the conditions shown in Table 1. With a commercially available toothbrush (Between, Zigzag Bristles, Regular Cut; manufactured by Lion Corporation) secured to a stroke-type abrasion tester (manufactured by TOKYO GIKEN INC.), an abrasion test was conducted under a 250 g load in 40,000 cycles with the coating surface of the specimen facing upward in the presence of a suspension of a commercially available tooth paste tooth paste (Dentor Clear MAX (Lion)/distilled water = 90/10 parts by mass). After the abrasion test, the glossiness of the specimen surface was measured with a gloss meter (VG-107 manufactured by Nippon Denshoku Industries Co., Ltd.) (n = 2), and the mean value was determined as a fraction relative to the glossiness of a mirror at 100%. The measurement was conducted at 60° angle.

In view of the gloss retention to maintain the same level of gloss as natural teeth over extended time periods, the preferred glossiness is 70% or more, more preferably 75% or more, even more preferably 80% or more, particularly preferably 85% or more.

Separately, surface roughness (arithmetic mean roughness Ra) was measured with a surface roughness meter Laser Focus Displacement Gauge LT-8100 (manufactured by Keyence) after the abrasion test, and mean values were calculated (n = 2).

The preferred arithmetic mean roughness Ra is 3.8 $\mu$m or less, more preferably 3.3 $\mu$m or less, even more preferably 2.8 $\mu$m or less, particularly preferably 2.3 $\mu$m or less.

The arithmetic mean roughness Ra may be, for example, 1.0 $\mu$m or more. In the table, "Surface roughness after wear ($\mu$m)" represents arithmetic mean roughness Ra ($\mu$m).

[Measurement of Shade Change]

The discotic zirconia base material prepared earlier was measured for chromaticity according to the L*a*b* evaluation system (JIS Z 8781-4:2013 Color Measurements - Part 4: CIE 1976 L*a*b* color space), using a spectrophotometer Crystaleye (manufactured by Olympus Corporation) in 7-band measurement mode with an LED light source, yielding L*0, a*0, b*0. Subsequently, a Clearfil® Ceramic® Primer Plus (manufactured by Kuraray Noritake Dental Inc.) was applied onto the discotic zirconia base material prepared earlier. After drying the material by blowing air, the composition prepared earlier was applied, and cured into a specimen under the conditions shown in Table 1.
The specimen was measured for chromaticity using the same method used for the chromaticity measurement of the zirconia base material, yielding L*1, a*1, b*1. The measured values were substituted in the formula below to determine ΔE* as an index of color change (n = 1).

$$\Delta E^* = \{(L^*1 - L^*0)^2 + (a^*1 - a^*0)^2 + (b^*1 - b^*0)^2\}^{1/2}$$

In view of the effect on the shade of the base material, the color difference ΔE* should preferably take values as small as possible, preferably 1.6 or less, more preferably 1.4 or less, even more preferably 1.2 or less, most preferably 1.0 or less.

[Table 1]

| Components (parts by mass) | Compounds | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|
| Urethane (meth)acrylate oligomer (A-1) | UV-1700B | 36.0 | 37.5 | 36.0 | 36.0 | 40.0 | 40.0 | 53.0 | 65.0 | 17.0 | 17.0 | 17.0 | 17.0 | 17.0 |
| | UA-306T | | | | | | | | | 19.0 | 19.0 | 19.0 | 19.0 | 19.0 |
| Polymerizable monomer (A-2) | SR454NS | 41.0 | 32.0 | 41.0 | 41.0 | 43.7 | 43.7 | 30.7 | 18.7 | 2.0 | 2.0 | 2.0 | 2.0 | 2.0 |
| | SR9003NS | | | | | | | | | 38.5 | 38.5 | 38.5 | 38.5 | 18.5 |
| | DPHA | | | | | | | | | | | | | 20.0 |
| | TMPTA | | | | | | | | | | | | | |
| | MMA | | | | | | | | | | | | | |
| | D2.6E | | | | | | | | | | | | | |
| | 3G | | | | | | | | | | | | | |
| | MDP | | | | | | | | | | | | | |
| Polyfunctional thiol (B) | MT-PE-1 | 19.5 | 26.5 | 19.5 | 19.5 | 12.8 | 12.8 | 12.8 | 12.8 | 20.0 | 20.0 | 20.0 | | |
| | 3-MPO | | | | | | | | | | | | 20.0 | 20.0 |
| Monofunctional thiol | 3-MPE | | | | | | | | | | | | | |
| Polysiloxane (C) | Composition C-1 | 1.0 | 1.5 | 1.0 | 1.0 | 1.0 | 1.0 | 1.0 | 1.0 | 1.0 | 1.0 | 1.0 | 1.0 | 1.0 |
| | KMP-702 | | | | | | | | | | | | | |
| Filler (E) | QSG-30 | 2.5 | 2.5 | 2.5 | 2.5 | 2.5 | | 2.5 | 2.5 | 2.5 | 2.5 | 2.5 | 2.5 | 2.5 |
| | R7200 | | | | | | 2.5 | | | | | | | |
| | Ba-1 | | | | | | | | | | | | | |
| | Ba-2 | | | | | | | | | | | | | |
| Polymerization initiator (D) | BAPO | 4.0 | 4.0 | 4.0 | 4.0 | 4.0 | 4.0 | 4.0 | 4.0 | 4.0 | | 1.4 | 4.0 | 1.4 |
| | Lucirin-TPO | | | | | | | | | | 5.0 | | | |
| | CQ | | | | | | | | | | | 1.4 | | 1.4 |
| Other | DMBE | | | | | | | | | | | 1.2 | | 1.2 |
| | DMEM | | | | | | | | | | | | | |

EP 4 364 718 A1

Table 1] Continued

| Components (parts by mass) | Compounds | Com. Ex. 1 | Com. Ex. 2 | Com. Ex. 3 | Com. Ex. 4 | Com. Ex. 5 | Com. Ex. 6 | Com. Ex. 7 |
|---|---|---|---|---|---|---|---|---|
| Urethane (meth)acrylate oligomer (A-1) | UV-1700B | | | | | | 36.0 | 46.0 |
| | UA-306T | | | | | | | |
| Polymerizable monomer (A-2) | SR454NS | | | | | | 41.0 | 50.5 |
| | SR9003NS | | | | | | | |
| | DPHA | 50.0 | 72.0 | | | | | |
| | TMPTA | 20.0 | | | | | | |
| | MMA | 30.0 | 22.0 | | | | | |
| | D2.6E | | | 14.0 | | | | |
| | 3G | | | 5.9 | | | | |
| | MDP | | 6.0 | | | | | |
| Polyfunctional thiol (B) | MT-PE-1 | | | | Seeseed N Color Coat Clear | Cerabien ZR FC Paste Stain Clear Glaze | | |
| | 3-MPO | | | | | | | |
| Monofunctional thiol | 3-MPE | | | | | | 19.5 | |
| Polysiloxane (C) | Composition C-1 | | | | | | 1.0 | 1.0 |
| | KMP-702 | | | 1.1 | | | | |
| Filler (E) | QSG-30 | | | | | | 2.5 | 2.5 |
| | R7200 | | | | | | | |
| | Ba-1 | | | 39.5 | | | | |
| | Ba-2 | | | 39.5 | | | | |
| Polymerization initiator (D) | BAPO | | | 4.0 | | | 4.0 | 4.0 |
| | Lucirin-TPO | 1.5 | | | | | | |
| | CQ | | 1.0 | | | | | |
| Other | DMBE | | 1.0 | | | | | |
| | DMEM | | 4.0 | | | | | |

22

Table 1] Continued

| | | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|---|
| Composition viscosity (mPa·s) | | | 100 | 500 | 100 | 100 | 100 | 100 | 100 | 100 | 100 | 100 | 100 | 100 | 500 |
| Composition operability | | | Good | Good | Good | Good | Good | Good | Good | Good | Good | Good | Good | Good | Good |
| Pencil hardness of cured film | | | 4H | 4H | 4H | 4H | 4H | 4H | 4H | 4H | 4H | 4H | 4H | 4H | 4H |
| Curing conditions | | | 10 sec.[1) | 10 sec.[1) | 20 sec.[1) | 30 sec.[1) | 20 sec.[1) | 20 sec.[1) | 20 sec.[1) | 20 sec.[1) | 20 sec.[1) | 20 sec.[1) | 20 sec.[1) | 20 sec.[1) | 20 sec.[1) |
| Abrasion resistance test simulating occlusion | Bovine enamel side | Wear volume (mm$^3$) | 0.053 | 0.050 | 0.024 | 0.018 | 0.056 | 0.061 | 0.055 | 0.050 | 0.022 | 0.025 | 0.021 | 0.088 | 0.072 |
| | | Wear diameter (mm) | 1.5 | 1.4 | 1.3 | 1.2 | 1.5 | 1.6 | 1.4 | 1.4 | 1.3 | 1.3 | 1.3 | 1.6 | 1.5 |
| | | Wear depth ($\mu$m) | 50.5 | 49.7 | 32.5 | 27.1 | 51.1 | 53.2 | 51.2 | 50.2 | 33.2 | 33.4 | 30.2 | 60.2 | 55.4 |
| | Coating layer side | Wear volume (mm$^3$) | 0.052 | 0.050 | 0.058 | 0.060 | 0.055 | 0.060 | 0.051 | 0.049 | 0.051 | 0.056 | 0.055 | 0.070 | 0.063 |
| Abrasion test | Glossiness after wear (%) | | 87.9 | 87.5 | 88.2 | 87.0 | 86.1 | 85.2 | 86.5 | 87.0 | 89.0 | 89.5 | 89.2 | 76.8 | 83.2 |
| | Surface roughness after wear ($\mu$m) | | 1.75 | 1.74 | 1.65 | 2.14 | 2.01 | 2.23 | 1.98 | 1.81 | 1.61 | 1.60 | 1.45 | 3.02 | 2.45 |
| Evaluation of shade change ($\Delta E^*$) | | | 0.77 | 0.74 | 0.25 | 0.69 | 0.32 | 0.45 | 0.38 | 0.41 | 0.25 | 0.19 | 1.38 | 0.78 | 1.34 |

Table 1] Continued

| | | | Com. Ex. 1 | Com. Ex. 2 | Com. Ex. 3 | Com. Ex. 4 | Com. Ex. 5 | Com. Ex. 6 | Com. Ex. 7 |
|---|---|---|---|---|---|---|---|---|---|
| Composition viscosity (mPa·s) | | | | | | | | | |
| Composition operability | | | Good | Good | Moderate | Moderate | Poor | Good | Good |
| Pencil hardness of cured film | | | | | | | | | |
| Curing conditions | | | 20 sec.[1] | 20 sec.[1] | 20 sec.[1] | 90 sec.[1] | 750°C, 1 min. | 20 sec.[1] | 20 sec.[1] |
| Abrasion resistance test simulating occlusion | Bovine enamel side | Wear volume ($mm^3$) | 0.028 | 0.037 | | 0.030 | 0.175 | 0.123 | 0.101 |
| | | Wear diameter (mm) | 1.3 | 1.3 | | 1.4 | 2.3 | 1.9 | 1.7 |
| | | Wear depth ($\mu$m) | 42.5 | 43.8 | | 35.2 | 76.4 | 73.3 | 70.1 |
| | Coating layer side | Wear volume ($mm^3$) | 0.087 | 0.081 | | 0.077 | 0.111 | 0.161 | 0.143 |
| Abrasion test | Glossiness after wear (%) | | 61.2 | 69.4 | 54.0 | 65.0 | 98.9 | 53.2 | 58.2 |
| | Surface roughness after wear ($\mu$m) | | 5.65 | 3.17 | 5.11 | 4.13 | 0.86 | 5.78 | 5.01 |
| Evaluation of shade change ($\Delta$E*) | | | 3.54 | 3.61 | | 3.92 | 1.78 | 6.89 | 5.23 |
| *1) Photoirradiation time | | | | | | | | | |

As shown in Table 1, Examples 1 to 13 were superior to Comparative Examples 1, 2, and 7 in terms of the gloss retention of the dental curable composition, and the effectiveness of shade change reduction.

Examples 1 to 13 showed excellent results in operability in all samples, and were superior to Comparative Example 3 in terms of operability and gloss retention.

Examples 1 to 13 were superior to the commercially available product of Comparative Example 4 in terms of operability, gloss retention, and the effectiveness of shade change reduction.

The polymerized and cured dental curable compositions of Examples 1 to 13 were shown to possess desirable abrasion resistance, such as small wear volumes, even in the oral cavity environment, compared to the commercially available product of Comparative Example 5.

Examples 1 to 13 were superior to Comparative Example 6 (containing a monofunctional thiol) in terms of the gloss retention of the dental curable composition and the effectiveness of shade change reduction.

The cured products of Examples 1 to 13 exhibited excellent abrasion resistance, and the abrasion resistance for the opposing tooth was also superior compared to Comparative Examples 5 to 7.

## Claims

1.   A dental curable composition comprising a polymerizable compound (A), a polyfunctional thiol (B) having two or more mercapto groups per molecule, a polysiloxane (C), and a polymerization initiator (D).

2.   The dental curable composition according to claim 1, wherein the polysiloxane (C) comprises a polysilsesquioxane (C-1).

3. The dental curable composition according to claim 1 or 2, wherein the polymerizable compound (A) comprises a urethane (meth)acrylate oligomer (A-1), and/or a polymerizable monomer (A-2).

4. The dental curable composition according to any one of claims 1 to 3, wherein the polyfunctional thiol (B) comprises a polyfunctional thiol having 2 to 4 mercapto groups per molecule.

5. The dental curable composition according to any one of claims 1 to 4, wherein the mercapto groups in the polyfunctional thiol (B) are bonded to secondary carbon atoms or tertiary carbon atoms.

6. The dental curable composition according to any one of claims 1 to 5, wherein the polysiloxane (C) is liquid at 25°C.

7. The dental curable composition according to any one of claims 1 to 6, wherein the polymerization initiator (D) comprises a photopolymerization initiator (D-1).

8. The dental curable composition according to any one of claims 1 to 7, which further comprises a filler (E).

9. The dental curable composition according to claim 8, wherein the filler (E) comprises a hydrophobic silica (E-1).

10. The dental curable composition according to claim 9, wherein the hydrophobic silica (E-1) has an average primary particle diameter of 5 to 100 nm.

11. The dental curable composition according to any one of claims 1 to 10, wherein the dental curable composition is a coating agent for coating a base material.

12. The dental curable composition according to claim 11, wherein the base material comprises at least one selected from the group consisting of zirconia, alumina, fused quartz, lithium silicate glass, an acrylic resin, and a composite material containing a polymer resin and an inorganic filler.

13. The dental curable composition according to claim 12, wherein the zirconia comprises zirconia, and a stabilizing agent capable of preventing a phase transformation of zirconia.

14. A dental prosthesis comprising a base material and a resin layer, wherein the resin layer comprises a polymerized and cured product of a dental curable composition comprising a polymerizable compound (A), a polyfunctional thiol (B) having two or more mercapto groups per molecule, a polysiloxane (C), and a polymerization initiator (D).

15. The dental prosthesis according to claim 14, wherein the base material comprises at least one selected from the group consisting of zirconia, alumina, fused quartz, lithium silicate glass, an acrylic resin, and a composite material containing a polymer resin and an inorganic filler.

16. The dental prosthesis according to claim 15, wherein the zirconia comprises zirconia, and a stabilizing agent capable of preventing a phase transformation of zirconia.

17. The dental prosthesis according to any one of claims 14 to 16, wherein the resin layer has a thickness of 1 $\mu$m to 500 $\mu$m.

18. The dental prosthesis according to any one of claims 14 to 17, wherein the polysiloxane (C) comprises a polysilsesquioxane (C-1).

19. The dental prosthesis according to any one of claims 14 to 18, wherein the polymerizable compound (A) comprises a urethane (meth)acrylate oligomer (A-1), and/or a polymerizable monomer (A-2).

20. The dental prosthesis according to any one of claims 14 to 19, wherein the polyfunctional thiol (B) comprises a polyfunctional thiol having 2 to 4 mercapto groups per molecule.

21. The dental prosthesis according to any one of claims 14 to 20, wherein the mercapto groups in the polyfunctional thiol (B) are bonded to secondary carbon atoms or tertiary carbon atoms.

22. The dental prosthesis according to any one of claims 14 to 21, wherein the polysiloxane (C) is liquid at 25°C.

23. The dental prosthesis according to any one of claims 14 to 22, wherein the polymerization initiator (D) comprises a photopolymerization initiator (D-1).

24. The dental prosthesis according to any one of claims 14 to 23, wherein the resin layer further comprises a filler (E).

25. The dental prosthesis according to claim 24, wherein the filler (E) comprises a hydrophobic silica (E-1).

26. The dental prosthesis according to claim 25, wherein the hydrophobic silica (E-1) has an average primary particle diameter of 5 to 100 nm.

## INTERNATIONAL SEARCH REPORT

International application No.

**PCT/JP2022/025853**

### A. CLASSIFICATION OF SUBJECT MATTER

*A61K 6/896*(2020.01)i; *A61K 6/20*(2020.01)i; *A61K 6/60*(2020.01)i; *A61K 6/64*(2020.01)i; *A61K 6/71*(2020.01)i; *A61K 6/76*(2020.01)i; *A61K 6/818*(2020.01)i; *A61K 6/887*(2020.01)i; *A61K 6/893*(2020.01)i
FI:   A61K6/896; A61K6/893; A61K6/887; A61K6/20; A61K6/60; A61K6/64; A61K6/71; A61K6/76; A61K6/818

According to International Patent Classification (IPC) or to both national classification and IPC

### B. FIELDS SEARCHED

Minimum documentation searched (classification system followed by classification symbols)

A61K6/896; A61K6/20; A61K6/60; A61K6/64; A61K6/71; A61K6/76; A61K6/818; A61K6/887; A61K6/893

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | JP 2016-153382 A (KURARAY NORITAKE DENTAL INC) 25 August 2016 (2016-08-25) claims, examples | 1-26 |
| Y | JP 64-26505 A (SHOWA DENKO KK) 27 January 1989 (1989-01-27) claims, examples | 1-26 |
| Y | JP 2019-116471 A (TOKUYAMA DENTAL CORP) 18 July 2019 (2019-07-18) claims, examples | 1-26 |
| Y | WO 2019/107323 A1 (MITSUI CHEMICALS, INC) 06 June 2019 (2019-06-06) claims, examples | 1-26 |

☐ Further documents are listed in the continuation of Box C.       ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **13 September 2022** | **20 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2022/025853**

| Patent document cited in search report | | | Publication date (day/month/year) | Patent family member(s) | | | Publication date (day/month/year) |
|---|---|---|---|---|---|---|---|
| JP | 2016-153382 | A | 25 August 2016 | (Family: none) | | | |
| JP | 64-26505 | A | 27 January 1989 | (Family: none) | | | |
| JP | 2019-116471 | A | 18 July 2019 | US | 2020/0330333 | A1 | |
| | | | | claims, examples | | | |
| | | | | WO | 2019/131788 | A1 | |
| | | | | EP | 3733150 | A1 | |
| | | | | CN | 111491600 | A | |
| WO | 2019/107323 | A1 | 06 June 2019 | US | 2021/0179745 | A1 | |
| | | | | claims, examples | | | |
| | | | | EP | 3719048 | A1 | |
| | | | | CN | 111225933 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018089312 A
- JP 2001149385 A
- JP 2005154312 A
- JP 2021054813 A
- JP 2016153382 A
- JP H0357916 B